(19) Europäisches Patentamt
European Patent Office
Office européen des brevets

(11) Publication number: **0 427 860 A1**

(12) **EUROPEAN PATENT APPLICATION published in accordance with Art. 158(3) EPC**

(21) Application number: 90904929.8

(51) Int. Cl.5: C07D 403/06, A61K 31/415

(22) Date of filing: 27.03.90

(86) International application number: PCT/JP90/00409

(87) International publication number: WO 90/12009 (18.10.90 90/24)

(30) Priority: 31.03.89 JP 81694/89

(43) Date of publication of application: 22.05.91 Bulletin 91/21

(84) Designated Contracting States: AT BE CH DE DK ES FR GB IT LI LU NL SE

(71) Applicant: Kyoto Pharmaceutical Industries, Ltd.
38, Nishinokyo Tsukinowa-cho Nakakyo-ku
Kyoto-shi Kyoto 604(JP)

(72) Inventor: MATSUI, Hiroshi
14-4, Mimatsu 3-chome Nara-shi
Nara 630(JP)
Inventor: KAMIYA, Syouzi 50-39, Kamikatsura Sannomiya-cho
Nishikyo-ku Kyoto-shi
Kyoto 615(JP)
Inventor: SHIRAHASE, Hiroaki 38-35, Imazato Kawahara
Nagaokakyo-shi
Kyoto 617(JP)
Inventor: MORIHISA, Rika 207, Villa Tamiaki 7, Kamiueno-cho Jigo Muko-shi
Kyoto 617(JP)
Inventor: NISHIMURA, Ken-ichi 14, Yamanouchi Miyamae-cho
Ukyo-ku Kyoto-shi
Kyoto 615(JP)
Inventor: KAKEYA, Nobuharu 10-16, Takadai 3-chome
Nagaokakyo-shi
Kyoto 617(JP)

(74) Representative: von Kreisler, Alek, Dipl.-Chem. et al
Patentanwälte Von Kreisler-Selting-Werner, Deichmannhaus am Hauptbahnhof
W-5000 Köln 1(DE)

(54) **NEW IMIDAZOLE DERIVATIVES, PRODUCTION THEREOF, AND USES THEREOF AS MEDICINES.**

(57) Imidazole derivatives of general formula (I) and pharmacologically acceptable salts thereof, wherein m and n are each an integer of 1 to 3, $R^1$ is hydrogen or ester residue, and the indoline skeleton may be substituted by at least one member selected from a $C_1$ to $C_{10}$ alkyl group and a $C_1$ to $C_{15}$ alkoxy group. These compounds have excellent pharmacological actions such as inhibition of lipid peroxide formation, inhibition of platelet agglutination caused by thromboxane $A_2$ synthetase inhibition, and vasodilation. Thus they are effectively used in preventing or treating asthma, thrombosis, embolism, arteriosclerosis, hypertension, hyperlipemia, cerebral apoplexy, cardiac infarction, dementia, diabetes, etc.

EP 0 427 860 A1

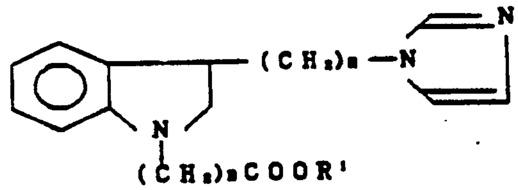 (I)

# NOVEL IMIDAZOLE DERIVATIVES, METHODS FOR THEIR PRODUCTION AND PHARMACEUTICAL USE THEREOF

[Field of Art]

The present invention relates to novel imidazole derivatives and their pharmacologically acceptable salts which are useful as pharmaceuticals, methods for their production and pharmaceutical use thereof.

[Background Art]

It has been known that imidazole derivatives have pharmacological activities such as inhibitory activities on platelet aggregation and vasodilative activities, etc. due to inhibitory activities on thromboxane $A_2$ synthase, and are useful for the prevention and treatment of circulatory organ disorders such as thrombosis, cerebral apoplexy, myocardial infarction, or the like. However, the present situation stands that the creation and pharmacological investigation of the derivatives are not entirely satisfactory.

An object of the present invention is to provide novel imidazole derivatives and their pharmacologically acceptable salts which exhibit markedly superior pharmacological activities such as inhibitory activities on platelet aggregation and vasodilative activities, etc. due to inhibitory activities on lipoperoxide production and on thromboxane $A_2$ synthase, and low toxicity, and methods for producing the same.

Another object of the invention is to provide agents for the prevention and treatment of diseases caused by thromboxane $A_2$ and/or lipoperoxide, such as asthma, thrombosis, embolism, arteriosclerosis, hypertension, hyperlipidemia, cerebral apoplexy, myocardial infarction, dementia, diabetes, and so on.

[Disclosure of the Invention]

The above-mentioned objects of the invention can be achieved by the following inventions ①, ②, ③ and ④.

① Imidazole derivatives [hereinafter referred to as imidazole derivatives (I)] of the formula

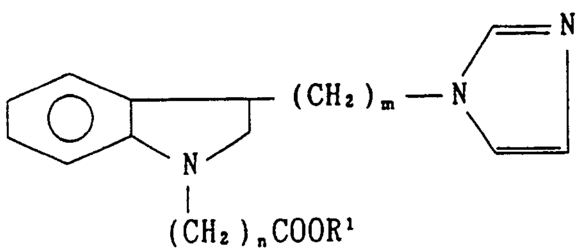

(I)

wherein $R^1$ is a hydrogen atom or an ester residue, m and n are respectively an integer from 1 to 3 and at least one substituent selected from alkyls having 1 to 10 carbon atoms and alkoxys having 1 to 15 carbon atoms may be comprised on the indoline skeleton and their pharmacologically acceptable salts.

② Methods for producing the imidazole derivatives or their pharmacologically acceptable salts, comprising (a) reacting compounds (II) to be described later and compounds (III) to be described later, (b) reacting compounds (II) and compounds(VIII) to be described later, (c) reacting compounds (X) to be described later and imidazole, or (d) reacting compounds (XI) to be described later and compounds (XII ) to be described later.

③ Agents for the prevention and treatment of diseases induced by thromboxane $A_2$, comprising the imidazole derivatives (I) and their pharmacologically acceptable salts as an active ingredient.

④ Agents for the prevention and treatment of diseases induced by lipoperoxide, comprising the imidazole derivatives (I) and their pharmacologically acceptable salts as an active ingredient.

In the invention, each group represents the following or the following may be examples thereof.

The alkyl having 1 to 10 carbon atoms may be a chain (straight or branched) or a ring, and the chain alkyl includes methyl, ethyl, n-propyl, iso-propyl, n-butyl, iso-butyl, sec-butyl, t-butyl, n-pentyl, iso-pentyl, neopentyl, hexyl, heptyl, octyl, nonyl, decyl, etc., with preference given to a chain alkyl having 3 to 8 carbon

atoms. A cyclic alkyl may be further comprised on the alkyl terminal, and examples thereof include cyclopropylmethyl, cyclobutylethyl, cyclopentylmethyl, or the like. The cyclic alkyl includes a cyclic alkyl having 3 to 6 carbon atoms such as cyclopropyl, cyclobutyl, cyclopentyl, cyclohexyl, or the like.

The alkoxy having 1 to 15 carbon atoms may be straight or branched, and is exemplified by methoxy, ethoxy, propoxy, iso-propoxy, butoxy, iso-butoxy, sec-butoxy, tert-butoxy, pentyloxy, hexyloxy, heptyloxy, octyloxy, nonyloxy, decyloxy, undecyloxy, dodecyloxy, tridecyloxy, tetradecyloxy, pentadecyloxy, etc., with preference given to an alkoxy having 5 to 9 carbon atoms.

As regards $R^1$, the ester residue combindly forms an ester with the carboxyl group in the formula (I), and is exemplified by a lower alkyl (e.g. an alkyl having 1 to 4 carbon atoms such as methyl, ethyl, n-propyl, iso-propyl, n-butyl, t-butyl, etc.), 1-alkanoyloxyalkyl, 1-alkoxycarbonyloxyalkyl, phthalidyl, 5-methyl-1,3-dioxolen-2-one-4-ylmethyl, or the like.

The carbon number of the alkanoyl moiety in the 1-alkanoyloxyalkyl is normally from 1 to 10, preferably from 1 to 7, and the carbon number of the alkyl moiety is normally from 1 to 3, preferably 1 or 2. Examples of such group include acetoxymethyl, propyonyloxymethyl, n-butyryloxymethyl, iso-butyryloxymethyl, pivaloyloxymethyl, n-valeryloxymethyl, 2-methylbutyryloxymethyl, iso-valeryloxymethyl, n-hexanoyloxymethyl, 3-methylvaleryloxymethyl, neohexanoyloxymethyl, 2-methylhexanoyloxymethyl, 2,2-dimethylbutyryloxymethyl, diethylacetoxymethyl, dipropylacetoxymethyl, 2,2-dimethylvaleryloxymethyl, neoheptanoyloxymethyl, cyclohexanecarbonyloxymethyl, cyclohexylacetoxymethyl, 1-acetoxyethyl, 1-propionyloxyethyl, 1-n-butyryloxyethyl, 1-iso-butyryloxyethyl, 1-n-valeryloxyethyl, 1-pivaloyloxyethyl, 1-iso-valeryloxyethyl, 1-n-hexanoyloxyethyl, 1-cyclohexanecarbonyloxyethyl, or the like.

The carbon number of the alkoxy moiety in the 1-alkoxycarbonyloxyalkyl is normally from 1 to 10, preferably from 1 to 7, and the carbon number of the alkyl moiety is normally from 1 to 3, preferably 1 or 2. Examples of such group include 1-methoxycarbonyloxyethyl, 1-ethoxycarbonyloxyethyl, 1-n-propoxycarbonyloxyethyl, 1-iso-propoxycarbonyloxyethyl, 1-n-butoxycarbonyloxyethyl, 1-tert-butoxycarbonyloxyethyl, 1-pentyloxycarbonyloxyethyl, 1-cyclohexyloxycarbonyloxyethyl, or the like.

The ester when $R^1$ is an ester residue is an ester such as acetoxymethyl, propionyloxymethyl, n-butyryloxymethyl, iso-valeryloxymethyl, pivaloyloxymethyl, 1-acetoxyethyl, 1-propionyloxyethyl, 1-iso-butyryloxyethyl, 1-n-valeryloxyethyl, 1-iso-valeryloxyethyl, 1-pivaloyloxyethyl, phthalidyl, 1-ethoxycarbonyloxyethyl, 1-cyclohexyloxycarbonyloxyethyl and 5-methyl-1,3-dioxolen-2-one-4-ylmethyl.

The number of the substituent(s) on the indoline skeleton which is (are) selected from alkyls having 1 to 10 carbon atoms and/or alkoxys having 1 to 15 carbon atoms is preferably none or from 1 to 3, more preferably none or 1 or 2, and the substituent(s) may be the same or different.

The imidazole derivatives (I) and their pharmacologically acceptable salts of the present invention possess optical isomers which are to be encompassed in the present invention.

The pharmacologically acceptable salts of the imidazole derivatives (I) of the present invention include acid addition salts with inorganic acids such as hydrochloric acid, hydrobromic acid, sulfuric acid, etc. and with organic acids such as acetic acid, fumaric acid, maleic acid, citric acid, tartaric acid, methanesulfonic acid, etc., salts with metals such as sodium, potassium, calcium, alminium, etc., salts with amino acids such as glycine, alanine, etc., and so on.

The imidazole derivatives (I) and their pharmacologically acceptable salts of the present invention possess extremely strong pharmacological activities.

Examples of the compounds of the present invention include the following:

o 3-(1H-Imidazol-1-ylmethyl)-1-indolinebutyric acid
o Ethyl 3-(1H-imidazol-1-ylmethyl)-1-indolinebutyrate
o 3-[2-(1H-Imidazol-1-yl)ethyl]-1-indolineacetic acid
o Ethyl 3-[2-(1H-imidazol-1-yl)ethyl]-1-indolineacetate
o 3-[2-(1H-Imidazol-1-yl)ethyl]-1-indolinepropionic acid
o Ethyl 3-[2-(1H-imidazol-1-yl)ethyl]-1-indolinepropionate
o 3-[2-(1H-Imidazol-1-yl)ethyl]-1-indolinebutyric acid
o Ethyl 3-[2-(1H-imidazol-1-yl)ethyl]-1-indolinebutyrate
o 3-[2-(1H-Imidazol-1-yl)propyl]-1-indolineacetic acid
o Ethyl 3-[2-(1H-imidazol-1-yl)propyl]-1-indolineacetate
o 3-[2-(1H-Imidazol-1-yl)ethyl]-5-methoxy-1-indolineacetic acid
o Ethyl 3-[2-(1H-imidazol-1-yl)ethyl]-5-methoxy-1-indolineacetate
o 3-[2-(1H-Imidazol-1-yl)ethyl]-5-methoxy-1-indolinepropionic acid
o Ethyl 3-[2-(1H-imidazol-1-yl)ethyl]-5-methoxy-1-indolinepropionate
o 5-Ethoxy-3-[2-(1H-imidazol-1-yl)ethyl]-1-indolineacetic acid
o Ethyl 5-ethoxy-3-[2-(1H-imidazol-1-yl)ethyl]-1-indolineacetate

- 5-Ethoxy-3-[2-(1H-imidazol-1-yl)ethyl]-1-indolinepropionic acid
- Ethyl 5-ethoxy-3-[2-(1H-imidazol-1-yl)ethyl]-1-indolinepropionate
- 3-[2-(1H-Imidazol-1-yl)ethyl]-5-propoxy-1-indolineacetic acid
- Ethyl 3-[2-(1H-imidazol-1-yl)ethyl]-5-propoxy-1-indolineacetate
- 3-[2-(1H-Imidazol-1-yl)ethyl]-5-propoxy-1-indolinepropionic acid
- Ethyl 3-[2-(1H-imidazol-1-yl)ethyl]-5-propoxy-1-indolinepropionate
- 5-Butoxy-3-[2-(1H-imidazol-1-yl)ethyl]-1-indolineacetic acid
- Ethyl 5-butoxy-3-[2-(1H-imidazol-1-yl)ethyl]-1-indolineacetate
- 5-Butoxy-3-[2-(1H-imidazol-1-yl)ethyl]-1-indolinepropionic acid
- Ethyl 5-butoxy-3-[2-(1H-imidazol-1-yl)ethyl]-1-indolinepropionate
- 3-[2-(1H-Imidazol-1-yl)ethyl]-5-pentyloxy-1-indolineacetic acid
- Ethyl 3-[2-(1H-imidazol-1-yl)ethyl]-5-pentyloxy-1-indolineacetate
- 3[2-(1H-Imidazol-1-yl)ethyl]-5-pentyloxy-1-indolinepropionic acid
- Ethyl 3-[2-(1H-imidazol-1-yl)ethyl]-5-pentyloxy-1-indolinepropionate
- 5-Hexyloxy-3-[2-(1H-imidazol-1-yl)ethyl]-1-indolineacetic acid
- Ethyl 5-hexyloxy-3-[2-(1H-imidazol-1-yl)ethyl]-1-indolineacetate
- 5-Hexyloxy-3-[2-(1H-imidazol-1-yl)ethyl]-1-indolinepropionic acid
- Ethyl 5-hexyloxy-3-[2-(1H-imidazol-1-yl)ethyl]-1-indolinepropionate
- 5-Heptyloxy-3-[2-(1H-imidazol-1-yl)ethyl]-1-indolineacetic acid
- Ethyl 5-heptyloxy-3-[2-(1H-imidazol-1-yl)ethyl]-1-indolineacetate
- 5-Heptyloxy-3-[2-(1H-imidazol-1-yl)ethyl]-1-indolinepropionic acid
- Ethyl 5-heptyloxy-3-[2-(1H-imidazol-1-yl)ethyl]-1-indolinepropionate
- 3-[2-(1H-Imidazol-1-yl)ethyl]-5-octyloxy-1-indolineacetic acid
- Ethyl 3-[2-(1H-imidazol-1-yl)ethyl]-5-octyloxy-1-indolineacetate
- 3-[2-(1H-Imidazol-1-yl)ethyl]-5-octyloxy-1-indolinepropionic acid
- Ethyl 3-[2-(1H-imidazol-1-yl)ethyl]-5-octyloxy-1-indolinepropionate
- 3-[(1H-Imidazol-1-yl)methyl]-5-octyloxy-1-indolinepropionic acid
- Ethyl 3-[(1H-imidazol-1-yl)methyl]-5-octyloxy-1-indolinepropionate
- 3-[2-(1H-Imidazol-1-yl)ethyl]-4-nonyloxy-1-indolineacetic acid
- Ethyl 3-[2-(1H-imidazol-1-yl)ethyl]-4-nonyloxy-1-indolineacetate
- 3-[2-(1H-Imidazol-1-yl)ethyl]-4-nonyloxy-1-indolinepropionic acid
- Ethyl 3-[2-(1H-imidazol-1-yl)ethyl]-4-nonyloxy-1-indolinepropionate
- 3-[(1H-Imidazol-1-yl)methyl]-4-nonyloxy-1-indolinepropionic acid
- Ethyl 3-[(1H-imidazol-1-yl)methyl]-4-nonyloxy-1-indolinepropionate
- 3-[2-(1H-Imidazol-1-yl)ethyl]-5-nonyloxy-1-indolineacetic acid
- Ethyl 3-[2-(1H-imidazol-1-yl)ethyl]-5-nonyloxy-1-indolineacetate
- 3-[2(1H-Imidazol-1-yl)ethyl]-5-nonyloxy-1-indolinepropionic acid
- Ethyl 3-[2-(1H-imidazol-1-yl)ethyl]-5-nonyloxy-1-indolinepropionate
- 3-[2-(1H-Imidazol-1-yl)ethyl]-6-nonyloxy-1-indolineacetic acid
- Ethyl 3-[2-(1H-imidazol-1-yl)ethyl]-6-nonyloxy-1-indolineacetate
- 3-[2-(1H-Imidazol-1-yl)ethyl]-6-nonyloxy-1-indolinepropionic acid
- Ethyl 3-[2-(1H-imidazol-1-yl)ethyl]-6-nonyloxy-1-indolinepropionate
- 3-[2-(1H-Imidazol-1-yl)ethyl]-7-nonyloxy-1-indolineacetic acid
- Ethyl 3-[2-(1H-imidazol-1-yl)ethyl]-7-nonyloxy-1-indolineacetate
- 3-[2-(1H-Imidazol-1-yl)ethyl]-7-nonyloxy-1-indolinepropionic acid
- Ethyl 3-[2-(1H-imidazol-1-yl)ethyl]-7-nonyloxy-1-indolinepropionate
- 5-Decyloxy-3-[2-(1H-imidazol-1-yl)ethyl]-1-indolineacetic acid
- Ethyl 5-decyloxy-3-[2-(1H-imidazol-1-yl)ethyl]-1-indolineacetate
- 5-Decyloxy-3-[2-(1H-imidazol-1-yl)ethyl]-1-indolinepropionic acid
- Ethyl 5-decyloxy-3-[2-(1H-imidazol-1-yl)ethyl]-1-indolinepropionate
- 5-Dodecyloxy-3-[2-(1H-imidazol-1-yl)ethyl]-1-indolineacetic acid
- Ethyl 5-dodecyloxy-3-[2-(1H-imidazol-1-yl)ethyl]-1-indolineacetate
- 5-Dodecyloxy-3-[2-(1H-imidazol-1-yl)ethyl]-1-indolinepropionic acid
- Ethyl 5-dodecyloxy-3-[2-(1H-imidazol-1-yl)ethyl]-1-indolinepropionate
- 5-Cyclohexyloxy-3-[2-(1H-imidazol-1-yl)ethyl]-1-indolineacetic acid
- Ethyl 5-cyclohexyloxy-3-[2-(1H-imidazol-1-yl)ethyl]-1-indolineacetate
- 5-Cyclohexyloxy-3-[2-(1H-imidazol-1-yl)ethyl]-1-indolinepropionic acid
- Ethyl 5-cyclohexyloxy-3-[2-(1H-imidazol-1-yl)ethyl]-1-indolinepropionate

- 5-Benzyloxy-3-[2-(1H-imidazol-1-yl)ethyl]-1-indolineacetic acid
- Ethyl 5-benzyloxy-3-[2-(1H-imidazol-1-yl)ethyl]-1-indolineacetate
- 5-Benzyloxy-3-[2-(1H-imidazol-1-yl)ethyl]-1-indolinepropionic acid
- Ethyl 5-benzyloxy-3-[2-(1H-imidazol-l-yl)ethyl]-l-indolinepropionate
- 3-[2-(1H-Imidazol-l-yl)ethyl]-5-phenethyloxy-l-indolineacetic acid
- Ethyl 3-[2-(lH-imidazol-l-yl)ethyl]-5-phenethyloxy-1-indolineacetate
- 3-[2-(lH-Imidazol-l-yl)ethyl]-5-phenethyloxy-1-indolinepropionic acid
- Ethyl 3-[2-(1H-imidazol-l-yl)ethyl]-5-phenethyloxy-1-indolinepropionate
- 5-Hydroxy-3-[2-(lH-imidazol-l-yl)ethyl]-l-indolineacetic acid
- Ethyl 5-hydroxy-3-[2-(1H-imidazol-l-yl)ethyl]-1-indolineacetate
- 5-Hydroxy-3-[2-(1H-imidazol-1-yl)ethyl]-1-indolinepropionic acid
- Ethyl 5-hydroxy-3-[2-(1H-imidazol-l-yl)ethyl]-1-indolinepropionate
- 5-Allyloxy-3-[2-(lH-imidazol-l-yl)ethyl]-1-indolineacetic acid
- Ethyl 5-allyloxy-3-[2-(lH-imidazol-1-yl)ethyl]-1-indolineacetate
- 5-Allyloxy-3-[2-(lH-imidazol-1-yl)ethyl]-l-indolinepropionic acid
- Ethyl 5-allyloxy-3-[2-(lH-imidazol-1-yl)ethyl]-1-indolinepropionate
- 3-[2-(1H-Imidazol-1-yl)ethyl]-5-methyl-l-indolineacetic acid
- Ethyl 3-[2-(1H-imidazol-1-yl)ethyl]-5-methyl-1-indolineacetate
- 3-[2-(lH-Imidazol-1-yl)ethyl]-5-methyl-l-indolinepropionic acid
- Ethyl 3-[2-(1H-imidazol-1-yl)ethyl]-5-methyl-1-indolinepropionate
- 3-[2-(1H-Imidazol-1-yl)ethyl]-5-propyl-1-indolineacetic acid
- Ethyl 3-[2-(1H-imidazol-1-yl)ethyl]-5-propyl-1-indolineacetate
- 3-[2-(1H-Imidazol-1-yl)ethyl]-5-propyl-1-indolinepropionic acid
- Ethyl 3-[2-(1H-imidazol-1-yl)ethyl]-5-propyl-1-indolinepropionate
- 3-[2-(1H-Imidazol-1-yl)ethyl]-5-butyl-1-indolineacetic acid
- Ethyl 3-[2-(1H-imidazol-1-yl)ethyl]-5-butyl-1-indolineacetate
- 3-[2-(1H-Imidazol-1-yl)ethyl]-5-butyl-1-indolinepropionic acid
- Ethyl 3-[2-(1H-imidazol-1-yl)ethyl]-5-butyl-1-indolinepropionate
- 3-[2-(1H-Imidazol-1-yl)ethyl]-5-pentyl-1-indolineacetic acid
- Ethyl 3-[2-(1H-imidazol-1-yl)ethyl]-5-pentyl-1-indolineacetate
- 3-[2-(1H-Imidazol-1-yl)ethyl]-5-pentyl-1-indolinepropionic acid
- Ethyl 3-[2-(1H-imidazol-1-yl)ethyl]-5-pentyl-1-indolinepropionate
- 3-[2-(1H-Imidazol-1-yl)ethyl]-5-hexyl-1-indolinepropionic acid
- Ethyl 3-[2-(1H-imidazol-1-yl)ethyl]-5-hexyl-1-indolinepropionate
- 3-[2-(1H-Imidazol-1-yl)ethyl]-5-octyl-1-indolineacetic acid
- Ethyl 3-[2-(1H-imidazol-1-yl)ethyl]-5-octyl-1-indolineacetate
- 3-[2-(1H-Imidazol-1-yl)ethyl]-5-octyl-1-indolinepropionic acid
- Ethyl 3-[2-(1H-imidazol-1-yl)ethyl]-5-octyl-1-indolinepropionate
- 5-Cyclopentyl-3-[2-(1H-imidazol-1-yl)ethyl]-1-indolineacetic acid
- Ethyl 5-cyclopentyl-3-[2-(1H-imidazol-1-yl)ethyl]-1-indolineacetate
- 5-Cyclopentyl-3-[2-(1H-imidazol-1-yl)ethyl]-1-indolinepropionic acid
- Ethyl 5-cyclopentyl-3-[2-(1H-imidazol-1-yl)ethyl]-1-indolinepropionate
- 3-[2-(1H-Imidazol-1-yl)ethyl]-5-methoxy-2-methyl-1-indolineacetic acid
- Ethyl 3-[2-(1H-imidazol-1-yl)ethyl]-5-methoxy-2-methyl-1-indolineacetate
- 3-[2-(1H-Imidazol-1-yl)ethyl]-5-methoxy-2-methyl-1-indolinepropionic acid
- Ethyl 3-[2-(1H-imidazol-1-yl)ethyl]-5-methoxy-2-methyl-1-indolinepropionate
- 3-[(1H-Imidazol-1-yl)methyl]-2-methyl-5-propoxy-1-indolineacetic acid
- Ethyl 3-[(1H-imidazol-1-yl)methyl]-2-methyl-5-propoxy-1-indolineacetate
- 3-[(1H-Imidazol-1-yl)methyl]-2-methyl-5-propoxy-1-indolinepropionic acid
- Ethyl 3-[(1H-imidazol-1-yl)methyl]-2-methyl-5-propoxy-1-indolinepropionate
- 3-[(1H-Imidazol-1-yl)methyl]-2-methyl-5-pentyloxy-1-indolineacetic acid
- Ethyl 3-[(1H-imidazol-1-yl)methyl]-2-methyl-5-pentyloxy-1-indolineacetate
- 3-[(1H-Imidazol-1-yl)methyl]-2-methyl-5-pentyloxy-1-indolinepropionic acid
- Ethyl 3-[(1H-imidazol-1-yl)methyl]-2-methyl-5-pentyloxy-1-indolinepropionate
- 3-[2-(1H-Imidazol-1-yl)ethyl]-6-methoxy-5-pentyloxy-1-indolinepropionic acid
- 3-[(1H-Imidazol-1-yl)methyl]-2-methyl-6-methoxy-5-pentyloxy-1-indolinepropionic acid
- 3-[(1H-Imidazol-1-yl)methyl]-5-pentyloxy-1-indolinebutyric acid
- Ethyl 3-[(1H-imidazol-1-yl)methyl]-5-pentyloxy-1-indolinebutyrate

o 3-[3-(1H-Imidazol-1-yl)propyl]-5-pentyloxy-1-indolineacetic acid
o Ethyl 3-[3-(1H-imidazol-1-yl)propyl]-2-methyl-5-pentyloxy-1-indolineacetate
o 3-[2-(1H-Imidazol-1-yl)ethyl]-4,6-dimethyl-5-pentyloxy-1-indolinepropionic acid
o 3-[2-(1H-Imidazol-1-yl)ethyl]-2,4,6-trimethyl-5-pentyloxy-1-indolinepropionic acid
o 3-[(1H-Imidazol-1-yl)methyl]-2,4,6-trimethyl-5-pentyloxy-1-indolinepropionicacid

and their pharmacologically acceptable salts.

The imidazole derivatives (I) and their pharmacologically acceptable salts of the present invention can be produced, for example, by the following methods.

Method 1

A method wherein a compound (II ) of the formula

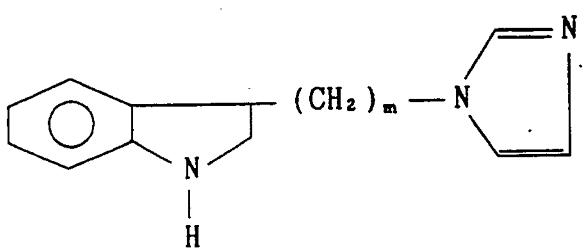

(II)

wherein m is an integer from 1 to 3, is reacted with a compound ( III) of the formula

$$X-(CH_2)_n-COOR^1 \quad (III)$$

wherein $R^1$ is as defined above and X is a group reactive with the amino group, preferably in the presence of a base.

As regards X, the group reactive with the amino group is halogen, alkanesulfonyloxy and arenesulfonyloxy, which are specifically exemplified by chlorine, bromine, iodine, methanesulfonyloxy, ethanesulfonyloxy, p-toluenesufonyloxy, or the like.

As the base, there may be mentioned sodium hydroxide, calcium hydroxide, sodium carbonate, potassium carbonate, pyridine, triethylamine, or the like.

The reaction proceeds preferably in the presence of a suitable solvent. Any solvent can be used as long as it does not interfere with the reaction, and examples thereof include alkanols such as methanol, ethanol, propanol, isopropanol, ethylene glycol, etc., ethers such as ethyl ether, dioxane, tetrahydrofuran, etc. , ketones such as acetone, methyl ethyl ketone, etc., water, pyridine, N,N-dimethylformamide, dimethylsulfoxide, acetic acid, benzene, acetonitile, chloroform, dichloromethane, ethyl acetate, or the like. The reaction temperature is normally between about -10 °C and about 150 °C, preferably between about 0 and about 120 °C. The reaction normally completes in 0.5 to 30 hours.

The starting compound (II) can be prepared by the following method.

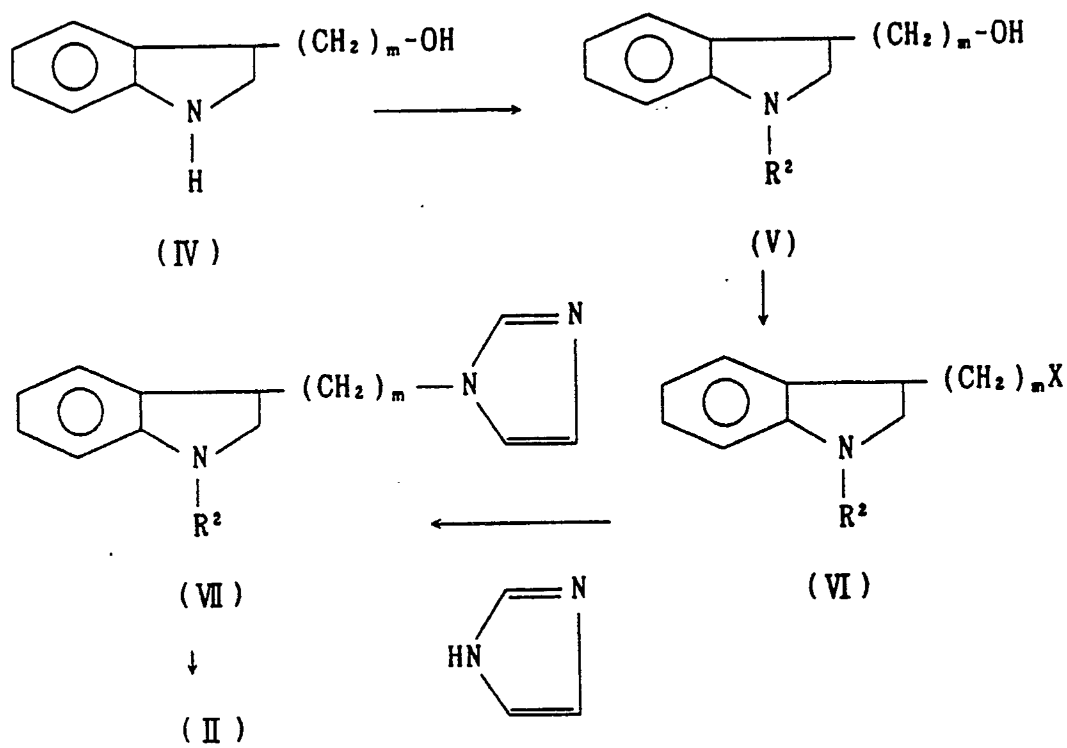

(IV)

(V)

(VII)

(VI)

(II)

wherein $R^2$ is an amino-protective group, and m and X are as defined above.

Compound (II) can be prepared by converting compounds (IV) to compounds (V) by a known method [e.g., G. W. Anderson, J. Am. Chem. Soc., *79* , 6180 (1957)], converting the hydroxyl group to an X-group to give compounds (VI) by a known method [e.g., J.G. Galzada, Org. Synth., *54*, 63 (1974)], and further reacting the same with imidazole to give compounds (VII), followed by deprotection.

As regards $R^2$, the protective group for the amino group may be known per se and examples thereof include t-butoxycarbonyl, ethoxycarbonyl, formyl, acetyl, trityl, benzyl, or the like.

Method 2

A method wherein the compound (II) mentioned above is reacted with a compound (VIII) of the formula

$$CH_2=CH-COOR^1 \qquad (VIII)$$

wherein $R^1$ is as defined above.

The reaction proceeds preferably without solvent or in the presence of a suitable solvent. Any solvent can be used as long as it does not interfere with the reaction, and examples thereof include methanol, ethanol, propanol, acetic acid, pyridine, DMF, or the like. A base may be used for accelerating the reaction. Examples of the base include NaOH, $K_2CO_3$, $Na_2CO_3$, $(C_2H_5)_3N$, or the like. The reaction temperature is between about 10°C and about 150°C, and the reaction time is normally from 1 to 25 hours. It is preferable that the compound (VIII) be used in an amount of 1 to 10 moles per 1 mole of the compound (II).

Method 3

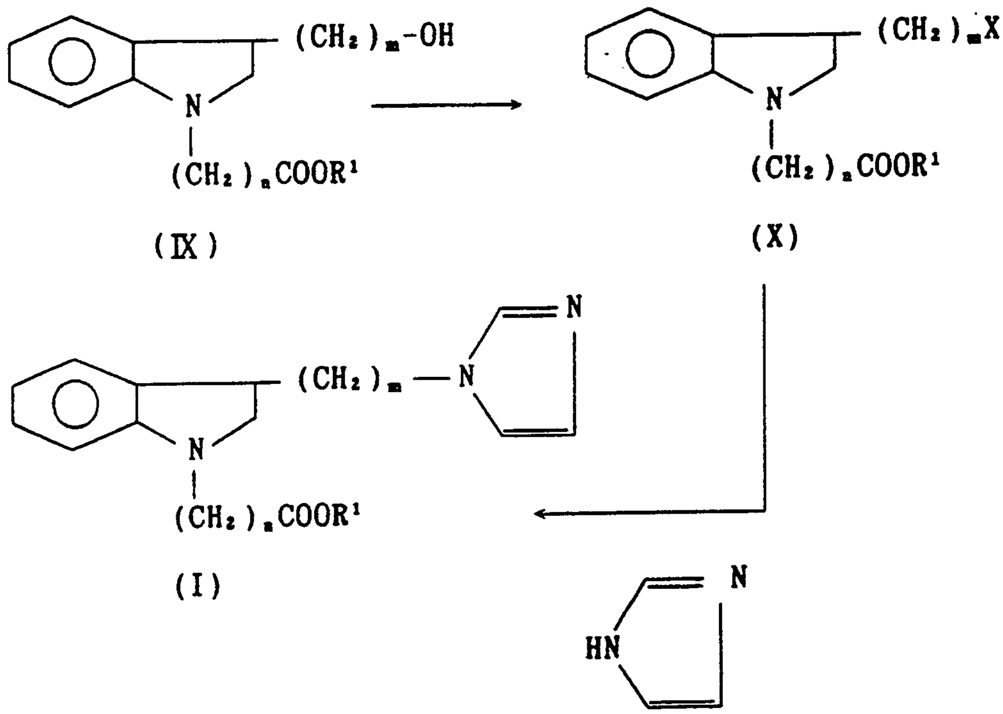

(IX) (X) (I)

wherein X, n, m and $R^1$ are as defined above.

This is a method wherein the hydroxyl group of compounds( IX) is converted to an X-group to give compounds (X) by a known method leg., J.G. Galzada, Org. Synth. , *54*, 63 (1974)], and further reacting the same with imidazole to give compounds (I).

The reaction between the compounds (X) and imidazole proceeds preferably in the presence of a suitable solvent. Any solvent can be used as long as it does not interfere with the reaction, and examples thereof include alkanols such as methanol, ethanol, propanol, isobutanol, etc., ethers such as ethyl ether, dioxane, tetrahydrofuran, etc., ketones such as acetone, methyl ethyl ketone, etc., water, pyridine, N,N-dimethylformamide, dimethylsulfoxide, acetic acid, benzene, acetonitile, chloroform, dichloromethane, ethyl acetate, or the like. Examples of the base include sodium hydroxide, calcium hydroxide, sodium carbonate, potassium carbonate, pyridine, triethylamine, or the like. The reaction proceeds at a temperature between room temperature and boiling point in 1 to 48 hours.

Method 4

Where compounds of the formula (I) possess an alkoxy group as a substituent on the indoline skeleton, the following method may be employed.

A method wherein a compound (XI) of the formula

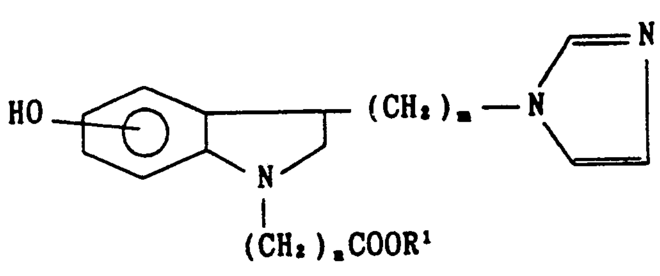

(XI)

wherein n, m and $R^1$ are as defined above, is reacted with a compound (XII ) of the formula

$$R^3\text{-}Y \qquad (XII)$$

wherein Y is a group reactive with the hydroxyl group and $R^3$ is an alkyl having 1 to 15 carbon atoms, in the presence of a base.

As regards Y, the group reactive with the hydroxyl group is halogen, alkanesulfonyloxy and arenesulfonyloxy, which are sepcifically exemplified by chlorine, bromine, iodine, methanesulfonyloxy, ethanesulfonyloxy, p-toluenesufonyloxy, or the like.

As the base, there may be mentioned sodium hydroxide, calcium hydroxide, sodium carbonate, potassium carbonate, pyridine, triethylamine, or the like.

The reaction proceeds preferably in the presence of a suitable solvent. Any solvent can be used as long as it does not interfere with the reaction, and examples thereof include alkanols such as methanol, ethanol, propanol, isopropanol, ethylene glycol, etc., ethers such as ethyl ether, dioxane, tetrahydrofuran, etc., ketones such as acetone, methyl ethyl ketone, etc., water, pyridine, N,N-dimethylformamide, dimethylsulfoxide, acetic acid, benzene, acetonitile, chloroform, dichloromethane, ethyl acetate, or the like. The reaction temperature is normally between about -10°C and about 150°C, preferably between about 0°C and about 120°C. The reaction normally completes in 0.5 to 30 hours.

The imidazole derivatives (I) and their pharmacologically acceptable salts are produced by a means known per se.

Further, the imidazole derivatives (I) having a carboxyl group or their salts may be converted to the ester compounds by a means known per se, and the ester compounds may be converted to the compounds having a carboxyl group or their salts by a means known per se.

The imidazole derivatives (I) and their pharmacologically acceptable salts can be obtained at optional purities by employing an isolation-purification method known per se such as extraction, chromatography, recrystallization, and so on.

The imidazole derivatives (I) and their pharmacologically acceptable salts exhibit inhibitory activities on platelet aggregation and vasodilative activities, etc. due to suppressive activities on lipoperoxide production and inhibitory activities on thromboxane $A_2$ synthase, to mammals such as humans, dogs, horses, rats, mice, etc., and in addition, show low toxicity. Thus, the compounds of the present invention are useful as agents for the prevention and treatment of asthma, thrombosis, embolism, arteriosclerosis, hypertension, myocardial infarction, angina pectoris, nephritis, cerebral apoplexy, ischemic cerebral circulatory disorders, and so on.

The imidazole derivatives (I) and their pharmacologically acceptable salts of the present invention can be formulated into, for example, tablets, sugar-coated agents, capsules, granules, powders, suppositories, injections, etc. in admixture with normally-employed additives by a known means, which can be administered orally or parenterally. The administration dose varies depending on the kind of diseases to be treated, symptoms, weight, age, sex, etc., but normally is 1 to 500 mg for adults per day for the treatment of asthma, thrombosis, embolism, hypertension, myocardial infarction, angina pectoris, nephritis, cerebral apoplexy, ischemic cerebral circulatory disorders, and so on.

Hereunder follow results of the pharmacological experiments showing effectiveness of the imidazole derivatives (I) and their pharmacologically acceptable salts of the present invention.

Experiment 1 (Inhibitory activities on platelet aggregation)

Platelet aggregation of platelet rich plasma (PRP) from rabbits was measured with the use of an NKK-hematracer (Niko Bioscience). That is, to PRP (200μ 1) was added a test drug or a solvent at 37°C while stirring at 1000 rpm, and an aggregation-inducing agent was added thereto 1 minute later, to measure the changes in transmittance caused by the platelet aggregation. Arachidonic acid (0.1 mM) was used as the aggregation-inducing agent.

Experiment 2 (Inhibitory activities on lipoperoxide production in rat brain)

To male rats weighing about 300 g was intraperitoneally administered 100 mg/kg of sodium pentobarbital and the rats were exsanguinated to death while anesthetized, after which the cerebrum was extracted. The extracted cerebrum was washed with ice-cooled physiological saline, dried gently, and weighed.

Thereto was added 5-fold amount by weight of a 50 mM phosphatesaline buffer (pH 7.4), which was then suspended by an ultradisperser under ice-cooling, followed by centrifugation at 1300 g and at 4°C for 10 minutes. The supernatant was 5-fold diluted with the above buffer, and used as a suspension for measurement.

The suspension to which the test drug had been added at 1/100 volume was incubated at 37°C for 30 minutes, and the amount of the resulting lipoperoxide was measured as an MDA yield. The assay of MDA was conducted by a modified method based on Smith et al., and the absorbance of the TBA reaction product at 532 nm was measured. The results are summarized in Table 1 (1).

Table 1 (1)

| Compound | Experiment 1 $10^{-6}$ M inhibition (%) | Experiment 2 $IC_{50}$ value |
|---|---|---|
| Example 1 | 40.3 | 93 × $10^{-6}$ M |
| 2 | 8.7 | |
| 5 | 20.3 | 25 × $10^{-6}$ M |
| 8 | 20.8 | 2.6 × $10^{-6}$ M |
| 9 | 28.3 | |
| 10 | 54.5 | 18.7× $10^{-6}$ M |
| 11 | 36.0 | 73 × $10^{-6}$ M |
| 12 | 19.7 | 4.8× $10^{-6}$ M |
| 13 | 10.4 | 21.9× $10^{-6}$ M |
| 14 | 14.5 | 6.0× $10^{-6}$ M |
| 15 | 1.6 | 24.8× $10^{-6}$ M |
| 16 | 2.1 | |
| 17 | 15.7 | 2.05× $10^{-6}$ M |
| 18 | 21.8 | |
| 19 | 3.6 | 19.4× $10^{-6}$ M |
| 20 | 45.2 | 2.05× $10^{-6}$ M |
| 21 | 5.5 | 1.84× $10^{-6}$ M |
| 22 | 37.0 | |
| 23 | 11.5 | 0.25× $10^{-6}$ M |
| 26 | 40.5 | |
| 27 | 27.0 | 0.26× $10^{-6}$ M |
| 29 | 12.1 | 0.24× $10^{-6}$ M |
| 30 | 28.4 | |
| 31 | 0.2 | 20.3× $10^{-6}$ M |
| OKY-046 | 4 | (-) |

Experiment 3 (Assay of $TXB_2$)

The test drug was orally administered to male rats, and 1 hour later, a blood sample was taken from ventral aorta via a polyethylene tube while anesthetized with ether. The blood taken was immediately incubated at 37°C for 90 minutes, and $TXB_2$ was extracted from the serum separated, followed by assay using a [$^3$H]RIA Kit. The results are summarized in Table 1 (2).

Table 1 (2)

| Compound | $TXB_2$ (ng/ml) |
|---|---|
| Control | 192.5 |
| Example 20 10 mg/kg | 5.1 |
| Example 21 10 mg/kg | 6.7 |
| Example 27 10 mg/kg | 4.8 |
| CV-4151 10 mg/kg | 11.0 |

Hereunder, the present invention is described in detail by working examples, to which the invention is not limited.

Example 1

Ethyl 3-[2-(1H-imidazol-1-yl)ethyl]-1-indolinepropionate

3-[2-(1H-Imidazol-1-yl)ethyl]indoline (30 g, 0.141 mol) is dissolved in acetic acid (600 ml), and ethyl acrylate (75 ml, 0.641 mol) is added thereto, followed by stirring at room temperature for 24 hours. The acetic acid is concentrated under reduced pressure, 2N sodium hydroxide is added to make the mixture alkaline, and the mixture is extracted with ethyl acetate. The organic layer is washed with saturated brine, dried over anhydrous sodium sulfate, after which the solvent is distilled off. The oily substance thus obtained is purified by silica gel column chromatography to give 38 g of the title compound as an oil (Yield 86%).

The properties of the compound thus obtained are as follows.
IR (Neat) $cm^{-1}$ : 2980, 2940, 1730, 1605
$^1H$-NMR ($CDCl_3$) δ ppm :
1.23 (3H, t, J=7.0Hz, $-CH_3$),

1.8 - 2.4 (2H, m, $-C\underline{H_2}CH_2$N 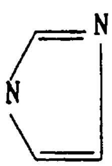 ),

2.55, (2H, t, J=6.0Hz, $-CH_2CO_2-$),

2.8 - 3.7 (5H, m, 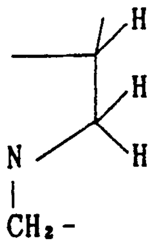 ), 3.8 - 4.5 (4H, m, $-CH_2$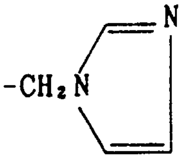 ,

$-CH_2CH_3$), 6.3 - 7.6 (7H, m, phenyl, -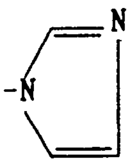 ).

Example 2

3-[2-(1H-Imidazol-1-yl)ethyl]-1-indolinepropionic acid

To ethyl 3-[2-(1H-imidazol-1-yl)ethyl]-1-indolinepropionate (38 g) is added 95% ethanol (600 ml) and allowed to dissolve. Thereto is added potassium hydroxide (molecular weight 56.11, 56 g), and the mixture is stirred at a temperature of 50 - 60 °C for 2 hours, after which the ethanol is distilled off under reduced pressure. Water (600 ml) is added thereto, and the mixture is washed 2 times with ethyl acetate (700 ml). The water layer (pH 9) is adjusted to pH 5 with 10% citric acid, saturated with sodium chloride, and is washed 3 times with chloroform (700 ml). The organic layer is dried over anhydrous sodium sulfate and the solvent is distilled off under reduced pressure. The oily residue is dissolved in ethanol (140 ml) under heating, allowed to cool, and kept standing in a refrigerater. The precipitated crystals are filtered off, and the filtrate is recrystallized from methanol (180 ml) to give 16.2 g of the title compound (Yield 40%), m.p. 138 - 141 °C.

The properties of the compound thus obtained are as follows.

IR (Nujol) $cm^{-1}$ : 3120, 2450, 1960, 1700, 1600

$^1H$-NMR ($DMSO-D_6$) $\delta$ ppm :

1.6 - 2.3 (2H, m, 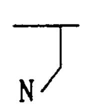 $CH_2$-),

2.46 (2H, t, J=6.0Hz, $-CH_2CO_2H$),

2.7 - 3.7 (5H, m, 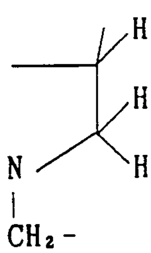 ), 4.01 (2H, t, J=7.0Hz, $-CH_2$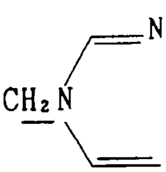 ),

6.15 (1H, br, $-CO_2H$),

6.3 - 7.3 (6H, m, phenyl, 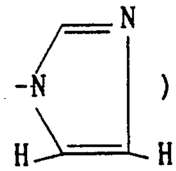 ),

7.64 (1H, s, 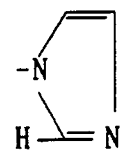 ).

Example 3

Ethyl 3-[2-(1H-imidazol-1-yl)ethyl]-1-indolineacetate

3-[2-(1H-Imidazol-1-yl)ethyl]indoline (974 mg, 4.57 mmol) is dissolved in acetone (20 ml), and thereto are added ethyl bromoacetate (molecular weight 167.00, 1.14 g, 6.83 mM) and potassium carbonate (molecular weight 138.21, 1.26 g, 9.12 mM), and the mixture is stirred for 17 hours. The acetone is distilled off under reduced pressure. Ethyl acetate (150 ml) is added thereto and the mixture is washed 3 times with water (100 ml) and once with saturated brine (100 ml). The mixture is dried over anhydrous sodium sulfate, and the solvent is distilled off under reduced pressure. The oily residue is purified by silica gel column chromatography to give 0.37 g of the title compound as an oil (Yield 27%).

The properties of the compound thus obtained are as follows.

IR (Neat) $cm^{-1}$ : 3380, 2940, 1745, 1605

$^1H$-NMR ($CDCl_3$) $\delta$ ppm :

1.26 (3H, t, J=7.0Hz, $-CH_3$), 1.9 - 2.4 (2H, m, $-\underline{CH_2}CH_2N<$),

3.0 - 3.8 (3H, m, 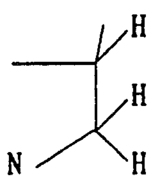 ), 3.86 (2H, s, $-CH_2CO_2-$), 4.0 (2H, t, J=7.0Hz, $-\underline{CH_2}$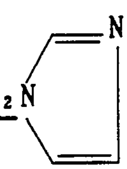 ),

4.16 (2H, q, J=7.0Hz, $-\underline{CH_2}CH_3-$),

6.2 - 7.6 (7H, m, phenyl, 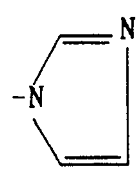 ).

Example 4

Sodium 3-[2-(1H-imidazol-1-yl)ethyl]-1-indolineacetic acid

Ethyl 3-[2-(1H-imidazol-1-yl)ethyl]-1-indolineacetate (852 mg, 2.85 mmol) is dissolved in 95% ethanol (20 ml), and thereto is added potassium hydroxide (molecular weight 56.11, 190 mg, 2.88 mM), and the mixture is stirred at 60 °C for 1 hour. Water (80 ml) and ethyl acetate (120 ml) are added for extraction and the water layer (pH 8) is adjusted to pH 5 with 10% citric acid, followed by washing with chloroform. The water layer is adjusted to pH 8 with a saturated aqueous solution of sodium bicarbonate and purified with the use of XAD-II column. The oily residue is solidified with a mixed solvent of chloroform-ethyl ether-methanol to give 270 mg of the title compound (Yield 32.3%).

The properties of the compound thus obtained are as follows.

IR (KBr) $cm^{-1}$ : 3400, 1605

$^1$H-NMR (DMSO-$D_6$) δ ppm :

1.5 - 2.4 (2H, m, $-\underline{CH_2}CH_2N<$),

2.6 - 3.9 (5H, m, 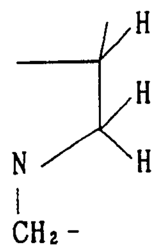 ),

4.02 (2H, t, J=7.0Hz, $-CH_2\underline{CH_2}N<$), 6.0 - 7.8 (7H, m, phenyl,

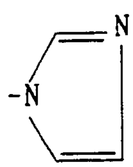 ).

Example 5

Ethyl 3-[2-(1H-imidazol-1-yl)ethyl]-1-indolineacetate

The title compound is obtained in the same manner as in Example 3. The properties of the obtained compound are as follows.

IR (Neat) $cm^{-1}$ 1730, 1608

$^1$H-NMR ($CDCl_3$) δ ppm :

1.23 (3H, t, J=7Hz, $-CH_2\underline{CH_3}$), 1.60 - 2.60 (6H, m, $>CH-\underline{CH_2}CH_2N<$, $-CH_2\underline{CH_2}\underline{CH_2}CO-$),

2.80 - 3.60 (5H, m, 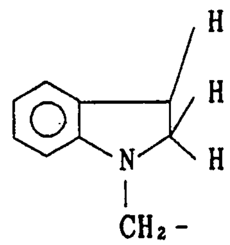), 4.03 (2H, t, J=8Hz, $-CH_2CH_2$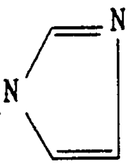), 4.10 (2H, q, J=7Hz, $-OCH_2CH_3$), 6.4 - 7.50 (7H, m, phenyl, -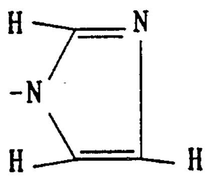).

Example 6

Ethyl 5-benzyloxy-3-[2-(1H-imidazol-1-yl)ethyl]-1-indolinepropionate oxalate

The title compound can be obtained in the same manner as in Example 1 using 5-benzyloxy-3-[2-(1H-imidazol-1-yl)ethyl]-indoline as a starting material.

The properties of the compound thus obtained are as follows (Yield 70%).
m.p. 111.5° C.
IR (Nujol) $cm^{-1}$ : 1735, 1625, 1490
$^1H$-NMR (DMSO-$D_6$)$\delta$ ppm :
1.17 (3H, t, J=7Hz, $OCH_2CH_3$), 1.6 - 2.4 (2H, m, $C_3$-$CH_2$ ), 2.52 (2H, m, $NCH_2CH_2CO$), 2.7 - 3.6 (5H, m, $C_2$-$H_2$, $C_3$-H, $NCH_2CH_2CO$), 5.45 (2H, s, $OCH_2$ $\phi$ ), 6.2 - 6.9 (3H, m, $C_{4,6,7}$-H), 7.31 (5H, s, $OCH_2$ $\phi$ ), 7.25, 7.48, 8.37 (3H, s, imidazole).

Example 7

Ethyl 5-hydroxy-3-[2-(1H-imidazol-1-yl)ethyl]-1-indolinepropionate oxalate

Oily ethyl 5-benzyloxy-3-[2-(1H-imidazol-1-yl)ethyl]-1-indolinepropionate (18.3 g) is dissolved in a mixture of annisole (92 ml) and trifluoroacetate (92 ml), and the mixture is stirred at a temperature of 70 - 75° C for 16 hours. The solvent is distilled off under reduced pressure, and thereto are added chloroform (300 ml) and water (400 ml). The water layer is separated and neutralized with saturated $NaHCO_3$, extracted with chloroform, washed with an aqueous solution of sodium chloride, and dried over $Na_2SO_4$. The chloroform is distilled off, and the oily substance thus obtained is dissolved in 99.5% ethanol (120 ml). Thereto is further added oxalic acid (5.76 g), and the mixture is made homogeneous, which is kept standing to give 12.1 g of the title compound, m.p. 111° C.
IR (Nujol) $cm^{-1}$ : 1735, 1630, 1500
$^1H$-NMR (DMSO-$D_6$)$\delta$ ppm :
1.19 (3H, t, J=7Hz, $OCH_2CH_3$), 1.6 - 2.4 (2H, m, $C_3$-$CH_2$), 2.52 (2H, m, $CH_2CO$), 2.7 - 3.6 (5H, m, $C_2$-$H_2$, $C_3$-H, $NCH_2CH_2CO$), 4.07 (2H, q, J=7Hz, $OCH_2CH_3$),

4.15 (2H, m, 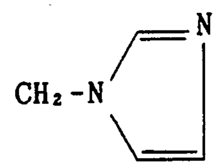 ), 6.28 (3H, s, $CO_2H$, OH), 6.2 - 6.7 (3H, m, phenyl), 7.36, 7.58, 8.56
|
$CO_2H$

(3H, s, imidazole).

Example 8

Ethyl 5-hexyloxy-3-[2-(1H-imidazol-1-yl)ethyl]-1-indolinepropionate

Ethyl 5-hydroxy-3-[2-(1H-imidazol-1-yl)ethyl]-1-indolinepropionate (2.7 g) is dissolved in 99.5% ethanol (45 ml), and potassium-tert-butoxide (0.92 g) is added thereto. After stirring the mixture at room temperature for 15 minutes, n-hexylbromide (1.49 g) is added thereto, followed by stirring at 90 °C for 7 hours. The solvent is distilled off, ethyl acetate and water are added thereto for extraction, and dried over $Na_2SO_4$, after which the ethyl acetate is distilled off. The residue thus obtained is purified by silica gel column chromatography to give 1.1 g of the oily compound (Yield 32%).
IR (Neat) $cm^{-1}$ : 1730, 1495
$^1$H-NMR ($CDCl_3$) $\delta$ ppm :
0.91 (3H, br-t, $-(CH_2)_5-CH_3$), 1.27 (3H, t, J=7Hz, $OCH_2CH_3$ ), 1.0 - 1.8 (8H, m, $OCH_2(CH_2)_4CH_3$), 1.9 - 2.3 (2H, m, $C_3-CH_2$), 2.57 (2H, t, J=6Hz, $-CH_2CO$), 2.8 - 3.4 (3H, m, $C_2-H_2$, $C_3$-H), 3.35 (2H, t, J=6Hz, >N-$CH_2CO$),

3.90 (2H, t, J=6Hz, 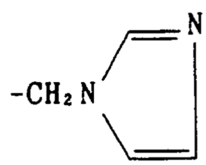 ),

4.15 (2H, q, J=7Hz, $-OCH_2CH_3$ ), 6.3 - 6.9 (3H, m, phenyl), 6.95, 7.09, 7.59 (3H, s, imidazole)

Example 9

5-Hexyloxy-3-[2-(1H-imidazol-1-yl)ethyl]-1-indolinepropionic acid

Ethyl 5-hexyloxy-3-[2-(1H-imidazol-1-yl)ethyl]-1-indolinepropionate ester (1.1 g) is dissolved in 95% ethanol (15 ml), and 2N-NaOH (4 ml) is added thereto, followed by stirring at room temperature for 3 hours. The ethanol is distilled off, and 5% citric acid is added thereto to adjust the solution to pH 4-5. The mixture is extracted with chloroform, washed with water, dried, and the solvent is distilled off. To the oily substance thus obtained is added 99.5% ethanol, and the mixture is kept standing to give 0.7 g of the title compound as crystals, m.p. 101 °C.
IR (Neat) $cm^{-1}$ : 1700, 1495
$^1$H-NMR (DMSO-$D_6$) $\delta$ ppm :
0.8 (3H, br-t, $-CH_2CH_3$), 1.0 -1.8 (8H, m, $-(CH_2)_4CH_3$), 1.8 - 2.3 (2H, m, $C_3-CH_2$ ), 2.46 (2H, t, J=6Hz, $-CH_2CO$), 2.65 - 3.60 (5H, m,

$C_2$-$H_2$, $C_3$-H, >N-$C\underline{H_2}CH_2$-), 3.85 (2H, t, J=6Hz, $OC\underline{H_2}CH_2$), 4.02 (2H, t, J=6Hz, 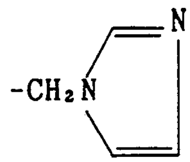 ), 4.70 (1H, br, $CO_2H$), 6.4 - 6.8 (3H, m, $C_4$-H, $C_6$-H, $C_7$-H), 6.91, 7.21, 7.70 (3H, s, imidazole)

Examples 10 - 33

Compounds represented by the formula

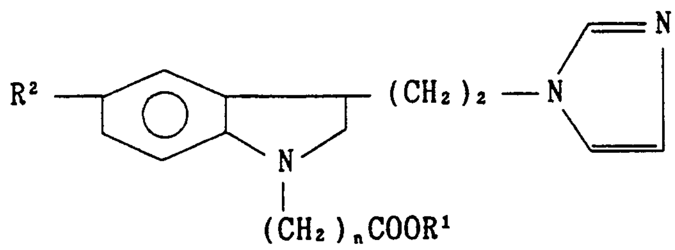

wherein n and $R^1$ are as defined above, are produced by the methods similar to those in the above-mentioned Examples. The IR and $^1$H-NMR of the obtained compounds are as shown in Table 2.

Examples 34 - 45

Compounds represented by the formula

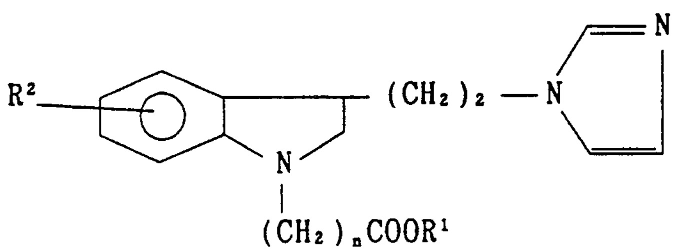

wherein n and $R^1$ are as defined above and there may be comprised at least one methyl group on the indoline skeleton, are produced by the methods similar to those in the above-mentioned Examples. The IR and $^1$H-NMR of the obtained compounds are as shown in Table 3.

Table 2 (1)

| Example No. | $R^1$ | $R^2$ | n | I R (Nujol) ($cm^{-1}$) | $^1H-NMR$ (DMSO-$D_6$; δ ppm) |
|---|---|---|---|---|---|
| 1 0 | $CH_3CH_2$ | $CH_3O$- | 2 | 1 7 1 5 | 1.24 (3H, t, J=7Hz, -$CH_2$<u>$CH_3$</u>),<br>1.8~2.4 (2H, m, $C_3$-C<u>$H_2$</u>-),<br>2.55 (2H, t, J=7Hz, -$CH_2CO$-),<br>2.8~3.6 (5H, m, $C_2$-$H_2$, $C_3$-H, >N-$CH_2$-),<br>3.72 (3H, s, -$OCH_3$),<br>3.7~4.4 (4H, m, OC<u>$H_2$</u>$CH_3$, -C<u>$H_2$</u>N 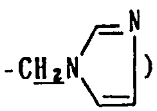 ),<br>6.2~6.8 (3H, m, phenyl),<br>6.8~7.6 (3H, m, imidazol)<br>(in $CDCl_3$) |
| 1 1 | H | $CH_3O$ | 2 | 1 7 0 0 | 1.5~2.4 (2H, m, $C_3$-$CH_2$-),<br>2.47 (2H, t, J=7Hz, -$CH_2CO$-),<br>2.60~3.50 (5H, m, $C_2$-$H_2$, $C_3$-H, >N-C<u>$H_2$</u>$CH_2CO$-),<br>3.63 (3H, s, -$OCH_3$),<br>4.02 (2H, t, J=7Hz, -C<u>$H_2$</u>N 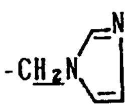 ),<br>6.2~6.8 (4H, m, phenyl , -$CO_2H$),<br>7.2~7.8 (3H, m, imidazol) |

Table 2 (2)

| Example No. | $R^1$ | $R^2$ | n | IR (Nujol) ($cm^{-1}$) | $^1H-NMR$ (DMSO-$D_6$, δ ppm) |
|---|---|---|---|---|---|
| 12 | $CH_3CH_2$ | $CH_3CH_2CH_2$- | 2 | 1730 | 0.91 (3H, t, J=6Hz, $-CH_2CH_2\underline{CH_3}$),<br>1.23 (3H, t, J=7Hz, $-OCH_2\underline{CH_3}$),<br>1.3 ~1.8 (2H, m, $CH_2\underline{CH_2}CH_3$),<br>2.0 (2H, m, $C_3$-$CH_2$),<br>2.48 (2H, t, J=8Hz, $C_5$-$CH_2$),<br>2.55 (2H, t, J=6Hz, $-CH_2CO-$),<br>3.05 (3H, m, $C_2$-$H_2$, $C_3$-H),<br>3.35 (2H, t, J=6Hz, $>N\underline{CH_2}CH_2CO$ ),<br>3.97 (2H, t, J=7Hz, $-CH_2-$ 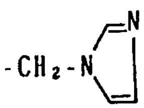 ),<br>4.10 (2H, q, J=7Hz, $O\underline{CH_2}CH_3$),<br>6.40 (1H, d, J=7Hz, $C_7$-H),<br>6.88 (2H, m, $C_4$-H, $C_6$-H),<br>6.80, 7.01 (2H, s, 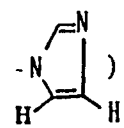 ),<br>7.42 (1H, s, 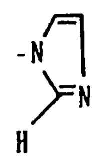 )<br>(in $CDCl_3$) |

Table 2 (3)

| Example | $R^1$ | $R^2$ | n | IR (Nujol) ($cm^{-1}$) | $^1H$-NMR (DMSO-$D_6$, δ ppm) |
|---|---|---|---|---|---|
| 13 | H | $CH_3CH_2CH_2$ | 2 | 1700 | 0.86 (3H, t, J=6Hz, $-CH_2C\underline{H_3}$),<br>1.5 (2H, m, $CH_2C\underline{H_2}CH_3$),<br>2.0 (2H, m, $C_3$-$CH_2$),<br>2.45 (4H, m, $C_5$-$CH_2$, $-CH_2C\underline{H_2}CO-$),<br>2.7 ~3.6 (5H, m, $C_2$-$H_2$, $C_3$-H, $>NC\underline{H_2}CH_2CO-$),<br>4.02 (2H, t, J=7Hz, $-CH_2$- 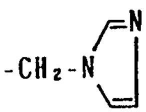 ),<br>4.85 (1H, br, $CO_2H$),<br>6.36 (1H, d, J=8Hz, $C_7$-H),<br>6.80 (2H, m, $C_4$-H, $C_6$-H),<br>6.82, 7.19 (2H, s, 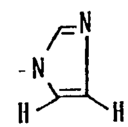 ),<br>7.65 (1H, s, 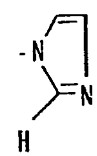 ) |

Table 2 (4)

| Example | $R^1$ | $R^2$ | n | IR (Nujol) ($cm^{-1}$) | $^1H-NMR$ ($DMSO-D_6$, $\delta$ ppm) |
|---|---|---|---|---|---|
| 14 | $CH_3CH_2$ | 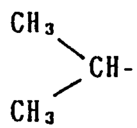 | 2 | 1730 | 1.19 (6H, d, J=7Hz, $-CH(C\underline{H_3})_2$),<br>1.25 (3H, t, J=7Hz, $-CH_2C\underline{H_3}$),<br>1.7 ~2.4 (2H, m, $C_3-CH_2$),<br>2.55 (2H, t, J=7Hz, $-C\underline{H_2}CO-$),<br>2.7 ~3.4 (4H, m, $C_3-CH$, $C_5-H$, $C_2-H_2$),<br>3.35 (2H, t, J=7Hz, $>NC\underline{H_2}CH_2CO$),<br>3.97 (2H, t, J=6Hz, $-CH_2-$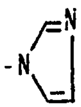),<br>4.09 (2H, q, J=7Hz, $OC\underline{H_2}CH_3$),<br>6.40 (1H, d, J=7Hz, $C_7-H$),<br>6.83 (1H, s, $C_4-H$),<br>6.90 (1H, d, J=8Hz, $C_6-H$),<br>6.83, 7.01 (2H, s, 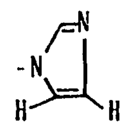),<br>7.41 (1H, s, 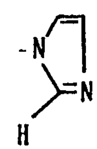)<br>(in $CDCl_3$) |

Table 2 (5)

| Example | $R^1$ | $R^2$ | n | IR (Nujol) ($cm^{-1}$) | $^1H-NMR$ (DMSO-$D_6$, $\delta$ ppm) |
|---|---|---|---|---|---|
| 15 | H | 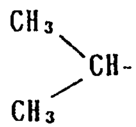 | 2 | 1700 | 1.15 (6H, d, J=7Hz, $CH(C\underline{H_3})_2$),<br>2.0 (2H, m, $C_3$-$CH_2$),<br>2.3 ~3.6 (8H, m, >$NCH_2CH_2CO$, $C_2$-$H_2$, $C_3$-H, CH ),<br>4.02 (2H, t, J=7Hz),<br>4.50 (1H, br, -$CO_2H$),<br>6.38 (1H, d, J=8Hz, $C_7$-H),<br>6.7 ~7.0 (2H, m, $C_6$-H, $C_4$-H),<br>6.88, 7.20 (2H, s, 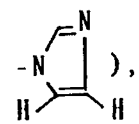 ),<br>7.66 (1H, s, 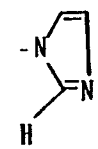 ) |

Table 2 (6)

| Example | $R^1$ | $R^2$ | n | IR (Nujol) ($cm^{-1}$) | $^1H-NMR$ (DMSO-$D_6$, δ ppm) |
|---|---|---|---|---|---|
| 16 oxalate | $CH_3CH_2$ | 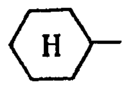 | 2 | 1730, 1500 | 1.18 (3H, t, J=7Hz, $OCH_2\underline{CH_3}$),<br>1.1 ~2.4 (13H, m, 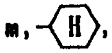, $C_3$-$CH_2$),<br>2.52 (2H, m, -$CH_2\underline{CH_2}$CO),<br>2.8 ~3.6 (5H, m, $C_2$-$H_2$, $C_3$-H, >$N\underline{CH_2}CH_2CO$),<br>3.98 (2H, t, J=7Hz, -$CH_2$-N 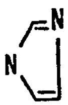),<br>4.15 (2H, q, J=7Hz, $O\underline{CH_2}CH_3$),<br>6.37 (1H, d, J=8Hz, $C_7$-H),<br>6.7 ~7.0 (2H, m, $C_4$-H, $C_6$-H),<br>6.98 (2H, s, $CO_2H$ / $CO_2H$),<br>7.42, 7.60 (2H, s, 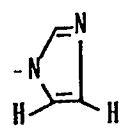),<br>8.72 (1H, s, 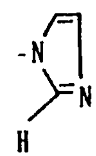) |

Table 2 (7)

| Example | $R^1$ | $R^2$ | n | I R (Nujol) ( $cm^{-1}$ ) | $^1H-NMR$ ( $DMSO-D_6$, δ ppm) |
|---|---|---|---|---|---|
| 1 7 | H | 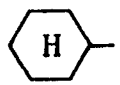 | 2 | 1 7 0 0 | 1.0 ~2.4 (13H, m, 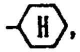, $C_3-CH_2$), 2.60 (2H, br-t, J=6Hz, $CH_2CO$), 2.9 ~4.2(7H, m, $C_2-H_2$, $C_3-H$, >$NC\underline{H_2}CH_2CO$, $CH_2N$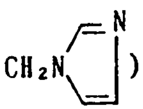), 6.45 (1H, d, J=8Hz, $C_7-H$), 6.90 (2H, m, $C_4-H$, $C_6-H$), 6.96, 6.80 (2H, s, -N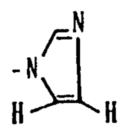), 7.57 (1H, s, -N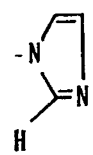), 8.48 (1H, br, $CO_2H$) (in $CDCl_3$) |

Table 2 (8)

| Example | $R^1$ | $R^2$ | n | IR (Nujol) ($cm^{-1}$) | $^1H-NMR$ (DMSO-$D_6$, δ ppm) |
|---|---|---|---|---|---|
| 18<br>oxalate | $CH_3CH_2$ | $CH_3(CH_2)_2O$ | 2 | 2800~<br>2300,<br>1735 | 0.96 (3H, t, J=7Hz, $-CH_2CH_2C\underline{H_3}$),<br>1.18 (3H, t, J=7Hz, $-OCH_2C\underline{H_3}$),<br>1.4 ~2.4 (4H, m, $-CH_2C\underline{H_2}CH_3$, $C_3-CH_2$),<br>2.55 (2H, t, J=7Hz, $-CH_2CO-$),<br>2.8 ~3.6 (5H, m, $C_2-H_2$, $C_3-H$, >N-$C\underline{H_2}$-),<br>3.6 ~4.5 (6H, m, $-OC\underline{H_2}CH_2-$, $-OC\underline{H_2}CH_3$, $-C\underline{H_2}N$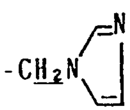 ),<br>6.40 (2H, s, $CO_2H$-$CO_2H$),<br>6.4 ~6.8 (3H, m, phenyl)<br>7.39, 7.60 (2H, s, 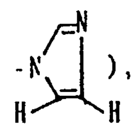 ),<br>8.60 (1H, s, 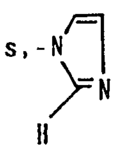 ) |

Table 2 (9)

| Example | $R^1$ | $R^2$ | n | IR (Nujol) ($cm^{-1}$) | $^1H-NMR$ ( $DMSO-D_6$, $\delta$ ppm) |
|---|---|---|---|---|---|
| 19 | H | $CH_3(CH_2)_2O$ | 2 | 1700 | 0.97 (3H, t, J=7Hz, $-CH_2CH_2C\underline{H_3}$), 1.3 ~2.7 (6H, m, $-CH_2C\underline{H_2}CH_3$, $C_3-CH_2-$, $-CH_2CO-$), 2.7 ~3.6 (5H, m, $C_2-H_2$, $C_3-H$, >N-$CH_2$-), 3.82 (2H, t, J=6Hz, OC $\underline{H_2}CH_2$-), 4.06 (2H, t, J=7Hz, $-C\underline{H_2}-N$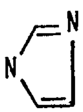 ), 4.60 (1H, br-s, $CO_2H$), 6.3 ~6.8 (3H, m, phenyl) 6.95, 7.25 (2H, s, -N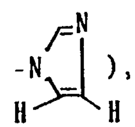 ), 7.72 (1H, s, -N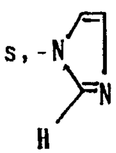 ) |

Table 2 (1)

| Example | $R^1$ | $R^2$ | n | IR (Nujol) ($cm^{-1}$) | $^1H$-NMR ($DMSO-D_6$, δ ppm) |
|---|---|---|---|---|---|
| 20 oxalate | $CH_3CH_2$ | $CH_3(CH_2)_4O$- | 2 | 1730, 1495 | 0.88 (3H, br-t, J=6Hz, $-CH_2C\underline{H_3}$),<br>1.16 (3H, t, J=7Hz, $OCH_2C\underline{H_3}$),<br>1.1 ~2.4 (8H, m, $-(C\underline{H_2})_3-CH_3$, $C_3-CH_2$),<br>2.51 (2H, t, J=6Hz, $-CH_2CO$),<br>2.7 ~3.5 (5H, m, $C_2-H_2$, $C_3-H$, >N-$C\underline{H_2}CH_2CO$),<br>3.80 (2H, t, J=6Hz, $C_5-OC\underline{H_2}$),<br>3.8 ~4.4 (2H, m, $-C\underline{H_2}$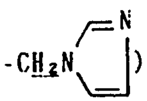),<br>4.05 (2H, q, J=7Hz, $-C\underline{H_2}CH_3$),<br>6.17 (2H, s, $CO_2H$),<br>$CO_2H$<br>6.35~6.70 (3H, m, phenyl)<br>7.31, 7.52 (2H, s, -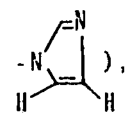),<br>8.55 (1H, s, -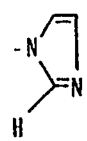) |

Table 2 (11)

| | $R^1$ | $R^2$ | n | IR (Nujol) ($cm^{-1}$) | $^1H-NMR$ ($DMSO-D_6$, δ ppm) |
|---|---|---|---|---|---|
| 21 | H | $CH_3(CH_2)_4O$ | 2 | 1700, 1492 | 0.89 (3H, br-t, $CH_2C\underline{H_3}$), 1.0 ~1.8 (6H, m, $-(C\underline{H_2})_3-CH_3$), 1.8 ~2.3 (2H, m, $C_3-CH_2$), 2.46 (2H, t, J=6Hz, $-CH_2CO$), 2.65~3.60 (5H, m, $C_2-H_2$, $C_3-H$, $>N-C\underline{H_2}CH_2-$), 3.81 (2H, t, J=6Hz, $OC\underline{H_2}CH_2$), 4.02 (2H, t, J=6Hz, $-CH_2N$ 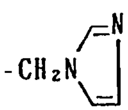 ), 4.70 (1H, br, $CO_2H$), 6.35 (1H, d, J=8Hz, $C_7-H$), 6.4 ~6.8 (2H, m, $C_4-H$, $C_6-H$), 6.88, 7.20, 7.67, (3H, s, imidazol) |

Table 2 (12)

| Example | $R^1$ | $R^2$ | n | I R (Nujol) ( $cm^{-1}$ ) | $^1H-NMR$ ( $DMSO-D_6$, $\delta$ ppm) |
|---|---|---|---|---|---|
| 22 oxalate | $CH_3CH_2$ | $CH_3(CH_2)_6O$ | 2 | 1740, 1640 | 0.88 (3H, br-t, $-(CH_2)_6-C\underline{H_3}$), 1.20 (3H, t, J=7Hz, $OCH_2C\underline{H_3}$), 1.0 ~2.3 (12H, m, $-(C\underline{H_2})_5$, $C_3-CH_2$), 2.55 (2H, m, $-CH_2CO$), 2.75~3.60 (5H, m, $C_2-H_2$, $C_3-H$, $>NC\underline{H_2}CH_2CO$), 3.85 (2H, br-t, $C_5-OC\underline{H_2}$), 4.07 (2H, q, J=7Hz, $\underset{\overset{\|}{O}}{C}OC\underline{H_2}CH_3$), 4.21 (2H, t, J=7Hz, $-CH_2N$ 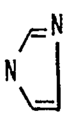 ), 6.14 (2H, s, $\underset{\underset{CO_2H}{\mid}}{CO_2H}$), 6.3 ~6.8 (3H, m, phenyl) 7.41, 7.62, 8.65, (3H, s, imidazol) |

Table 2 (13)

| Example | $R^1$ | $R^2$ | n | I R (Nujol) ( $cm^{-1}$ ) | $^1H-NMR$ ( $DMSO-D_6$, $\delta$ ppm) |
|---|---|---|---|---|---|
| 2 3 | H | $CH_3(CH_2)_6O$ | 2 | 1 7 0 0, 1 4 9 5 | 0.88 (3H, br-t, -$C\underline{H_3}$),<br>1.32 (10H, br-s, -$(CH_2)_5$-),<br>2.0 (2H, m, $C_3$-$CH_2$),<br>2.50 (2H, m, -$CH_2CO$),<br>2.7 ~3.6 (5H, m, $C_2$-$H_2$, $C_3$-H, >N-$CH_2CH_2CO$),<br>3.85 (2H, t, J=6Hz, $C_5$-$OCH_2$),<br>4.08 (2H, t, J=7Hz, -$CH_2N$ 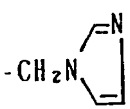 ),<br>5.10 (1H, br, $CO_2H$),<br>6.3 ~6.8 (3H, m, phenyl)<br>6.97, 7.28, 7.80, (3H, s, imidazol) |

Table 2 (14)

| Example | $R^1$ | $R^2$ | n | I R (Nujol) ( $cm^{-1}$ ) | $^1H-NMR$ ( $DMSO-D_6$, δ ppm) |
|---|---|---|---|---|---|
| 2 4<br>oxalate | $CH_3CH_2$ | $CH_3(CH_2)_7O$ | 2 | 1 7 3 0,<br>1 4 9 5 | 0.85 (3H, br-t, $(CH_2)_7C\underline{H_3}$),<br>1.18 (3H, t, J=7Hz, $OCH_2C\underline{H_3}$),<br>1.0 ~2.3 (14H, m, $-(C\underline{H_2})_6$, $C_3-CH_2$),<br>2.52 (2H, t, J=6Hz, $-CH_2CO-$),<br>2.7 ~3.6 (5H, m, $C_2-H_2$, $C_3-H$, $>NC\underline{H_2}CH_2CO$),<br>4.07 (2H, q, J=7Hz, $OC\underline{H_2}CH_3$),<br>4.0 ~4.6 (2H, m, $CH_2N$ 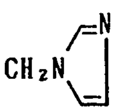 ),<br>5.50 (2H, s, $CO_2H$ \| $CO_2H$),<br>6.3 ~6.9 (3H, m, phenyl),<br>7.35, 7.55, 8.55, (3H, s, imidazol) |

Table 2 (15)

| Example | $R^1$ | $R^2$ | n | IR (Nujol) ($cm^{-1}$) | $^1H-NMR$ ($DMSO-D_6$, $\delta$ ppm) |
|---|---|---|---|---|---|
| 25 | H | $CH_3(CH_2)_7O$ | 2 | 1695, 1490 | 0.87 (3H, br-t, $CH_3$), 1.0 ~1.8 (12H, m, $(CH_2)_6$), 2.48 (2H, br-t, $CH_2CO$), 2.6 ~3.6 (5H, m, $C_2$-$H_2$, $C_3$-H, $NC\underline{H_2}CH_2CO$), 3.84 (2H, t, J=6Hz, $C_5$-$OCH_2$), 5.50 (1H, br, $CO_2H$), 6.3 ~6.8 (3H, m, phenyl), 6.95, 7.25, 7.75, (3H, s, imidazol) |

Table 2 (16)

| Example | $R^1$ | $R^2$ | n | IR (Nujol) ($cm^{-1}$) | $^1H-NMR$ ($DMSO-D_6$, $\delta$ ppm) |
|---|---|---|---|---|---|
| 26<br>oxalate | $CH_3CH_2$ | $CH_3(CH_2)_8O$ | 2 | 1730,<br>1495 | 0.85 (3H, br-t, J=6Hz, $-(CH_2)_8C\underline{H_3}$),<br>1.17 (3H, t, J=7Hz, $OCH_2CH_3$),<br>1.0 ~1.8 (14H, m, $-(C\underline{H_2})_7-$),<br>1.8 ~2.3 (2H, m, $C_3-CH_2$),<br>2.51 (2H, t, J=6Hz, $-CH_2CO$),<br>2.7 ~3.6 (5H, m, $C_2-H_2$, $C_3-H$, $>NC\underline{H_2}CH_2CO$),<br>3.82 (2H, t, J=6Hz, $OC\underline{H_2}-$),<br>4.07 (2H, q, J=7Hz, $OC\underline{H_2}CH_3$),<br>4.0 ~4.6 (2H, m, $CH_2-$ 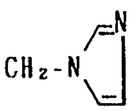 ),<br>5.10 (2H, s, 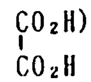),<br>6.3 ~6.9 (3H, m, phenyl),<br>7.31, 7.53, 8.45, (3H, s, imidazol) |

Table 2 (17)

| Example | $R^1$ | $R^2$ | n | IR (Nujol) ($cm^{-1}$) | $^1H-NMR$ (DMSO-$D_6$, $\delta$ ppm) |
|---|---|---|---|---|---|
| 27 | H | $CH_3(CH_2)_8O$ | 2 | 1695, 1490 | 0.86 (3H, br-t, -$(CH_2)_8$-$C\underline{H_3}$),<br>1.0 ~1.8 (14H, m, -$(C\underline{H_2})_7$-),<br>1.8 ~2.3 (2H, m, $C_3$-$CH_2$),<br>2.46 (2H, br-t, -$CH_2CO$),<br>2.6 ~3.6 (5H, m, $C_2$-$H_2$, $C_3$-H, >N-$C\underline{H_2}CH_2CO$),<br>3.83 (2H, t, J=6Hz, $OCH_2$-),<br>4.04 (2H, t, J=6Hz, -$CH_2$-N 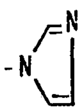 ),<br>4.4 (1H, br, $CO_2H$),<br>6.3 ~6.8 (3H, m, phenyl),<br>6.91, 7.22, 7.68, (3H, s, imidazol) |

Table 2 (18)

| Example | $R^1$ | $R^2$ | n | IR (Nujol) ($cm^{-1}$) | $^1H-NMR$ ($DMSO-D_6$, δ ppm) |
|---|---|---|---|---|---|
| 28 | $CH_3CH_2$ | $CH_3(CH_2)_{11}O$ | 2 | 1730, 1495 | 0.89 (3H, br-t, $-(CH_2)_{11}\underline{CH_3}$),<br>1.23 (3H, t, J=7Hz, $OCH_2\underline{CH_3}$),<br>1.0 ~1.8 (20H, m, $-(CH_2)_{10}-$),<br>1.8 ~2.4 (2H, m, $C_3-CH_2$),<br>2.57 (2H, t, J=6Hz, $-CH_2CO$),<br>2.8 ~3.4 (3H, m, $C_2-H_2$, $C_3-H$),<br>3.35 (2H, t, J=6Hz, $>N-\underline{CH_2}CH_2CO$),<br>3.89 (2H, t, J=6Hz, $C_5-OCH_2$),<br>4.0 (2H, m, $-CH_2-$ 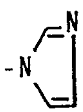 ),<br>4.05 (2H, q, J=7Hz, $-\underset{\underset{O}{\parallel}}{C}OCH_2-$),<br>6.3 ~6.9 (3H, m, phenyl),<br>6.95, 7.07, 7.52, (3H, s, imidazol)<br>(in $CDCl_3$) |

Table 2 (19)

| Example | $R^1$ | $R^2$ | n | IR (Nujol) ($cm^{-1}$) | $^1H-NMR$ (DMSO-$D_6$, $\delta$ ppm) |
|---|---|---|---|---|---|
| 29 | H | $CH_3(CH_2)_{11}O$ | 2 | 1700, 1490 | 0.86 (3H, br-t, $-(CH_2)_{11}C\underline{H_3}$), 1.0 ~1.9 (20H, m, $-(CH_2)_{10}-$), 1.9 ~2.4 (2H, m, $C_3-CH_2$), 2.50 (2H, m, $-CH_2CO$), 2.7 ~3.6 (5H, m, $C_2-H_2$, $C_3-H$, $>NC\underline{H_2}CH_2CO$), 3.85 (2H, br-t, $C_5-OCH_2$), 4.06 (2H, br-t, $-CH_2-$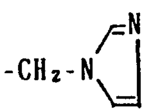 ), 6.3 ~6.9 (3H, m, phenyl), 6.96, 7.35, 7.75, (3H, s, imidazol) |

Table 2 (20)

| Example | $R^1$ | $R^2$ | n | I R (Nujol) ( $cm^{-1}$ ) | $^1H-NMR$ ( $DMSO-D_6$, $\delta$ ppm) |
|---|---|---|---|---|---|
| 3 0 oxalate | $CH_3CH_2$ | $CH_2=CHCH_2O$ | 2 | 1 7 3 0, 1 6 2 0 | 1.18 (3H, t, J=6Hz, $OCH_2C\underline{H_3}$),<br>2.10 (2H, m, $C_3-CH_2$),<br>2.52 (2H, m, $-CH_2CO-$),<br>2.7 ~3.6 (5H, m, $C_2-H_2$, $C_3-H$, >N-$C\underline{H_2}CH_2CO$),<br>3.99 (2H, t, J=7Hz, $-CH_2N$ 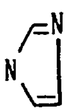 ),<br>4.10 (2H, q, J=6Hz, $OC\underline{H_2}CH_3$),<br>4.45 (2H, d, J=5Hz, $=CH-CH_2O$),<br>5.0 ~6.2 (3H, m, $CH_2=CH-$),<br>6.39 (1H, d, J=8Hz, $C_7-H$),<br>6.70 (2H, m, $C_4-H$, $C_6-H$),<br>7.25, 7.46, 8.38 (3H, s, imidazol-H)<br>9.20 (2H, br, $CO_2H$ \| $CO_2H$) |

Table 2 (21)

| Example | $R^1$ | $R^2$ | n | IR (Nujol) ($cm^{-1}$) | $^1H-NMR$ (DMSO-$D_6$, δ ppm) |
|---|---|---|---|---|---|
| 31 | H | $CH_2=CHCH_2O$ | 2 | 1700 | 2.0 (2H, m, $C_3$-$CH_2$),<br>2.45 (2H, t, J=6Hz, -$CH_2$CO-),<br>2.6 ~3.7 (5H, m, $C_2$-$H_2$, $C_3$-H, >N-$C\underline{H_2}CH_2$CO),<br>4.00 (2H, t, J=7Hz, -$CH_2$-N 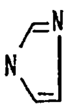 ),<br>4.45 (2H, d, J=6Hz, =CH-$C\underline{H_2}$O),<br>5.0 ~6.3 (3H, m, $CH_2$=CH-),<br>6.38 (1H, d, J=8Hz, $C_7$-H),<br>6.59 (1H, d, J=2Hz, $C_4$-H),<br>6.80 (1H, d, d, J=2Hz, 8Hz, $C_6$-H),<br>7.05, 7.35, 7.90 (3H, s, imidazol-H)<br>8.50 (1H, br, $CO_2$H) |

Table 2 (22)

| Example | $R^1$ | $R^2$ | n | IR (Nujol) ($cm^{-1}$) | $^1H-NMR$ (DMSO-$D_6$, δ ppm) |
|---|---|---|---|---|---|
| 32 | $CH_3CH_2$ | $CH_3(CH_2)_5$- | 2 | 1730 | 0.91 (3H, br-t, $-CH_2CH_2C\underline{H_3}$),<br>1.23 (3H, t, J=7Hz, $-OCH_2C\underline{H_3}$),<br>1.3 ~1.8 (8H, m, $CH_2(C\underline{H_2})_4CH_3$),<br>2.0 (2H, m, $C_3-CH_2$),<br>2.48 (2H, t, J=8Hz, $C_5-H_2$),<br>2.55 (2H, t, J=6Hz, $-CH_2-CO$),<br>3.05 (3H, m, $C_2-H_2$, $C_3-H$),<br>3.35 (2H, t, J=6Hz, $>NC\underline{H_2}CH_2CO-$),<br>3.97 (2H, t, J=7Hz, $-CH_2-$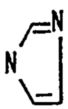 ),<br>4.10 (2H, q, J=7Hz, $-OC\underline{H_2}CH_3$),<br>6.40 (1H, d, J=7Hz, $C_7-H$),<br>6.88 (2H, m, $C_4-H$, $C_6-H$),<br>6.80, 7.01 (2H, s, 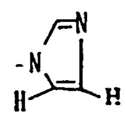 )<br>7.42 (1H, s, 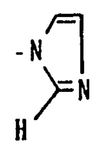 ) (in $CDCl_3$) |

Table 2 (23)

| Example | $R^1$ | $R^2$ | n | IR (Nujol) ($cm^{-1}$) | $^1H-NMR$ (DMSO-$D_6$, $\delta$ ppm) |
|---|---|---|---|---|---|
| 33 | H | $CH_3(CH_2)_5-$ | 2 | 1700 | 0.86 (3H, t, J=6Hz, $-CH_2C\underline{H_3}$),<br>1.5 (2H, m, $-CH_2(C\underline{H_2})_4CH_3$),<br>2.0 (2H, m, $C_3-CH_2$),<br>2.45 (4H, m, $C_5-CH_2$, $-CH_2C\underline{H_2}CO-$),<br>2.7 ~3.6 (5H, m, $C_2-H_2$, $C_3-H$, $>NC\underline{H_2}CH_2CO-$),<br>4.02 (2H, t, J=7Hz, $-CH_2-$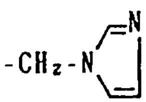),<br>4.85 (1H, br, $-CO_2H$),<br>6.36 (1H, d, J=8Hz, $C_7-H$),<br>6.80 (2H, m, $C_4-H$, $C_6-H$),<br>6.82, 7.19 (2H, s, 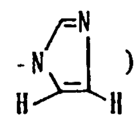)<br>7.65 (1H, s, 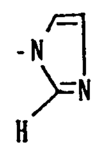) |

Table 3 (1)

| Example | $R^1$ | $R^2$ | n | I R (Nujol) ( $cm^{-1}$ ) | $^1H-NMR$ ( DMSO-$D_6$, $\delta$ ppm ) |
|---|---|---|---|---|---|
| 3 4 oxalate | 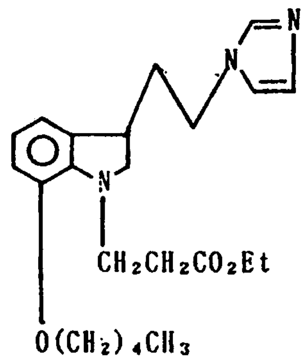 | | | 1 7 3 0 | 0.88 (3H, br-t, J=6Hz, $-CH_2C\underline{H_3}$),<br>1.16 (3H, t, J=7Hz, $-OCH_2C\underline{H_3}$),<br>1.1 ~2.4 (8H, m, $-(C\underline{H_2})_3-CH_3$, $C_3-CH_2$),<br>2.51 (2H, t, J=6Hz, $-CH_2CO-$),<br>2.7 ~3.5 (5H, m, $C_2-H_2$, $C_3-H$, $>NC\underline{H_2}CH_2CO-$),<br>3.80 (2H, t, J=6Hz, $C_5-OCH_2$),<br>3.8 ~4.4 (2H, m, $-CH_2-$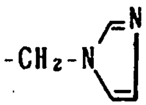 ),<br>4.05 (2H, q, J=7Hz, $-OC\underline{H_2}CH_3$),<br>6.17 (2H, s, 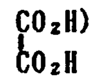),<br>6.45~7.50 (3H, m, phenyl),<br>7.31, 7.52 (2H, s, 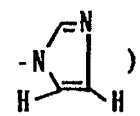 )<br>8.55 (1H, s, 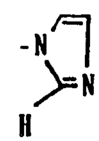 ) |

Table 3 (2)

| Example | $R^1$ | $R^2$ | n | IR (Nujol) ($cm^{-1}$) | $^1H-NMR$ ($DMSO-D_6$, $\delta$ ppm) |
|---|---|---|---|---|---|
| 35 | 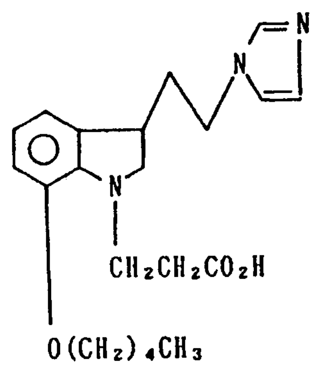 | | | 1700 | 0.89 (3H, br-t, $-CH_2C\underline{H_3}$), 1.0 ~1.8 (6H, m, $-(C\underline{H_2})_3-CH_3$), 1.8 ~2.3 (2H, m, $C_3-CH_2$), 2.46 (2H, t, J=6Hz, $-CH_2CO-$), 2.65~3.60 (5H, m, $C_2-H_2$, $C_3-H$, >N-$C\underline{H_2}CH_2-$), 3.81 (2H, t, J=6Hz, $-OC\underline{H_2}CH_2-$), 4.02 (2H, t, J=6Hz, $-C\underline{H_2}$-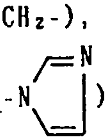), 4.70 (1H, br, $-CO_2H$), 6.45~7.45 (3H, m, phenyl), 6.88, 7.20, 7.67 (3H, s, imidazol) |

Table 3(3)

| Example | $R^1$ | $R^2$ | n | IR (Nujol) ($cm^{-1}$) | $^1H$-NMR ($DMSO-D_6$, δ ppm) |
|---|---|---|---|---|---|
| 36 oxalate | 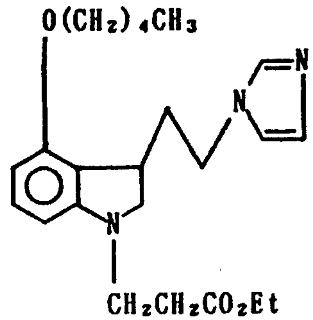 | | | 1730 | 0.88 (3H, br-t, J=6Hz, $-CH_2C\underline{H_3}$),<br>1.16 (3H, t, J=7Hz, $-OCH_2C\underline{H_3}$),<br>1.1 ~2.4 (8H, m, $-(C\underline{H_2})_3-CH_3$, $C_3-CH_2$),<br>2.51 (2H, t, J=6Hz, $-CH_2CO-$),<br>2.7 ~3.5 (5H, m, $C_2-H_2$, $C_3-H$, $>NC\underline{H_2}CH_2CO-$),<br>3.80 (2H, t, J=6Hz, $C_5-OCH_2$),<br>3.8 ~4.4 (2H, m, $-CH_2-$ 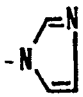 ),<br>4.05 (2H, q, J=7Hz, $-OC\underline{H_2}CH_3$),<br>6.17 (2H, s, $CO_2H$-$CO_2H$),<br>6.35~7.40 (3H, m, phenyl),<br>7.31, 7.52 (2H, s, 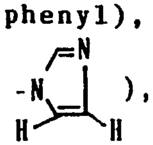 ),<br>8.55 (1H, s, 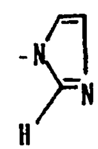 ) |

Table 3 (4)

| Example | $R^1$ | $R^2$ | n | IR (Nujol) ($cm^{-1}$) | $^1H-NMR$ (DMSO-$D_6$, $\delta$ ppm) |
|---|---|---|---|---|---|
| 37 | 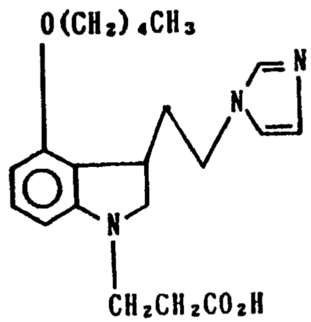 | | | 1700 | 0.89 (3H, br-t, -$CH_2\underline{CH_3}$), 1.0 ~1.8 (6H, m, -$(\underline{CH_2})_3$-$CH_3$), 1.8 ~2.3 (2H, m, $C_3$-$CH_2$), 2.46 (2H, t, J=6Hz, -$CH_2CO$-), 2.65~3.60 (5H, m, $C_2$-$H_2$, $C_3$-H, >N-$\underline{CH_2}CH_2$-), 3.81 (2H, t, J=6Hz, -O$\underline{CH_2}CH_2$-), 4.02 (2H, t, J=6Hz, -$\underline{CH_2}$-N 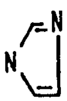 ), 4.70 (1H, br, -$CO_2H$), 6.35~7.41 (3H, m, phenyl), 6.88, 7.20, 7.67 (3H, s, imidazol) |

Table 3 (5)

| Example | $R^1$ | $R^2$ | n | IR (Nujol) ($cm^{-1}$) | $^1H-NMR$ ($DMSO-D_6$, δ ppm) |
|---|---|---|---|---|---|
| 38 oxalate | 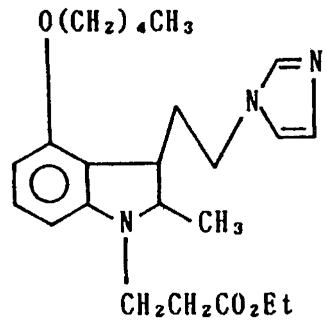 | | | 1730 | 0.88 (3H, br-t, $-CH_2C\underline{H_3}$),<br>1.16 (3H, t, J=7Hz, $-OCH_2C\underline{H_3}$),<br>1.35 (3H, d, J=6Hz, $C_2-CH_3$),<br>1.1 ~2.4 (8H, m, $-(C\underline{H_2})_3-CH_3$, $C_3-CH_2$),<br>2.51 (2H, t, J=6Hz, $-CH_2CO-$),<br>2.7 ~3.5 (4H, m, $C_2$-H, $C_3$-H, $>NC\underline{H_2}CH_2CO-$),<br>3.80 (2H, t, J=6Hz, $C_5-OCH_2$),<br>3.8 ~4.4 (2H, m, $-CH_2-$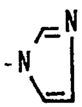 ),<br>4.05 (2H, q, J=7Hz, $-OC\underline{H_2}CH_3$),<br>6.17 (2H, s, $CO_2H$), $CO_2H$<br>6.35~6.70 (3H, m, phenyl),<br>7.31, 7.52 (2H, s, 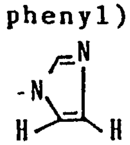 )<br>8.55 (1H, s, 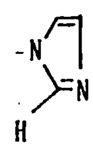 ) |

Table 3 (6)

| Example | R¹ | R² | n | IR (Nujol) (cm⁻¹) | ¹H-NMR (DMSO-D₆, δ ppm) |
|---|---|---|---|---|---|
| 39 | 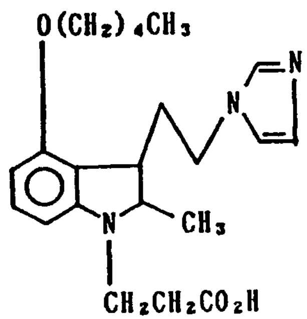 | | | 1700 | 0.89 (3H, br-t, $-CH_2C\underline{H_3}$), 1.35 (3H, d, J=6Hz, $C_2-CH_3$), 1.0 ~1.8 (6H, m, $-(C\underline{H_2})_3-CH_3$), 1.8 ~2.3 (2H, m, $C_3-CH_2$), 2.46 (2H, t, J=6Hz, $-CH_2CO-$), 2.65~3.60 (4H, m, $C_2-H_2$, $C_3-H$, $>N-C\underline{H_2}CH_2-$), 3.81 (2H, t, J=6Hz, $-OC\underline{H_2}CH_2-$), 4.02 (2H, t, J=6Hz, $-C\underline{H_2}-N$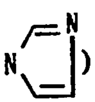), 4.70 (1H, br, $-CO_2H$), 6.35 (1H, d, J=8Hz, $C_7-H$), 6.4 ~6.8 (2H, m, $C_4-H$, $C_6-H$), 6.88, 7.20, 7.67 (3H, s, imidazol) |

Table 3 (7)

| Example | $R^1$ | $R^2$ | n | IR (Nujol) ($cm^{-1}$) | $^1H-NMR$ ($DMSO-D_6$, δ ppm) |
|---|---|---|---|---|---|
| 40 oxalate | 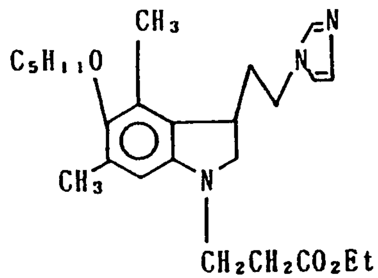 | | | 1730 | 0.88 (3H, br-t, J=6Hz, $-CH_2C\underline{H_3}$),<br>1.16 (3H, t, J=7Hz, $-OCH_2C\underline{H_3}$),<br>1.1 ~2.4 (8H, m, $-(C\underline{H_2})_3-CH_3$, $C_3-CH_2$),<br>2.41, 2.42 (6H, s, $C_4-CH_3$, $C_6-C\underline{H_3}$),<br>2.51 (2H, t, J=6Hz, $-CH_2CO-$),<br>2.7 ~3.5 (5H, m, $C_2-H_2$, $C_3-H$, $>NC\underline{H_2}CH_2CO-$),<br>3.80 (2H, t, J=6Hz, $C_5-OCH_2$),<br>3.8 ~4.4 (2H, m, $CH_2-$ 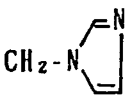 ),<br>4.05 (2H, q, J=7Hz, $-OC\underline{H_2}CH_3$),<br>6.17 (2H, s, $CO_2H$), $CO_2H$<br>6.45 (1H, s, phenyl),<br>7.31, 7.52 (2H, s, 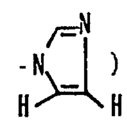 )<br>8.55 (1H, s, 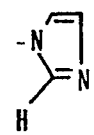 ) |

Table 3 (8)

| Example | $R^1$ | $R^2$ | n | I R (Nujol) ( $cm^{-1}$) | $^1H-NMR$ ( DMSO-$D_6$, δ ppm ) |
|---|---|---|---|---|---|
| 4 1 | 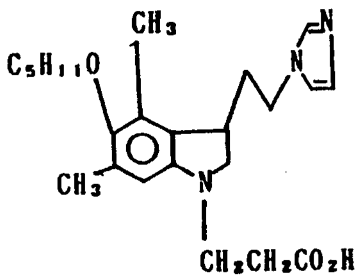 | | | 1 7 0 0 | 0.89 (3H, br-t, $-CH_2CH_3$),<br>1.0 ~1.8 (6H, m, $-(CH_2)_3-CH_3$),<br>1.8 ~2.3 (2H, m, $C_3-CH_2$),<br>2.41, 2.42 (6H, s, $C_4-CH_3$, $C_6-CH_3$),<br>2.46 (2H, t, J=6Hz, $-CH_2CO-$),<br>2.65~3.60 (5H, m, $C_2-H_2$, $C_3-H$, $>N-CH_2CH_2-$),<br>3.81 (2H, t, J=6Hz, $-OCH_2CH_2-$),<br>4.02 (2H, t, J=6Hz, $-CH_2-$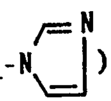),<br>4.70 (1H, br, $-CO_2H$),<br>6.45 (1H, s, $C_7-H$),<br>6.88, 7.20, 7.67 (3H, s, imidazol) |

Table 3 (9)

| Example | $R^1$ | $R^2$ | n | I R (Nujol) ( $cm^{-1}$) | $^1H-NMR$ ( DMSO-$D_6$, $\delta$ ppm ) |
|---|---|---|---|---|---|
| 4 2 oxalate | 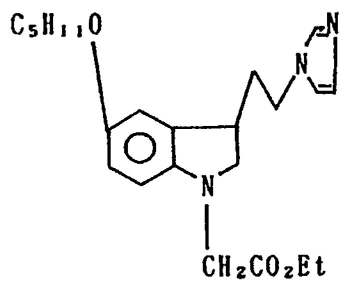 | | | 1 7 3 0 | 0.88 (3H, br-t, J=6Hz, $-CH_2C\underline{H_3}$),<br>1.16 (3H, t, J=7Hz, $-OCH_2C\underline{H_3}$),<br>1.1 ~2.4 (8H, m, $-(C\underline{H_2})_3-CH_3$, $C_3-CH_2$),<br>2.7 ~3.5 (3H, m, $C_2-H_2$, $C_3-H$),<br>3.80 (2H, t, J=6Hz, $C_5-OCH_2$),<br>3.86 (2H, s, $-CH_2CO-$),<br>4.04 (2H, t, J=7Hz, $-CH_2-$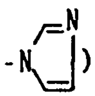),<br>4.05 (2H, q, J=7Hz, $-OC\underline{H_2}CH_3$),<br>6.17 (2H, s, $CO_2H$ / $CO_2H$),<br>6.35~6.70 (3H, m, phenyl),<br>7.32, 7.54 (2H, s, 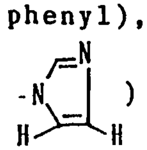 )<br>8.57 (1H, s, 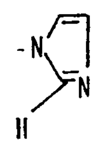 ) |

Table 3 (10)

| Example | $R^1$ | $R^2$ | n | IR (Nujol) ($cm^{-1}$) | $^1H-NMR$ (DMSO-$D_6$, δ ppm) |
|---|---|---|---|---|---|
| 43 | 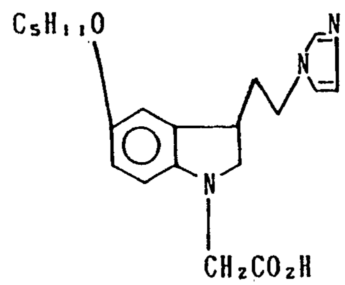 | | | 1700 | 0.89 (3H, br-t, -$CH_2C\underline{H_3}$),<br>1.0 ~1.8 (6H, m, -$(C\underline{H_2})_3$-$CH_3$),<br>1.8 ~2.3 (2H, m, $C_3$-$CH_2$),<br>2.65~3.60 (3H, m, $C_2$-$H_2$, $C_3$-H),<br>3.86 (2H, s, -$CH_2CO$-),<br>3.81 (2H, t, J=6Hz, -$OC\underline{H_2}CH_2$-),<br>4.02 (2H, t, J=6Hz, -$C\underline{H_2}$-N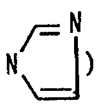),<br>4.90 (1H, br, -$CO_2H$),<br>6.35 (1H, d, J=8Hz, $C_7$-H),<br>6.4 ~6.8 (2H, m, $C_4$-H, $C_6$-H),<br>6.91, 7.23, 7.68 (3H, s, imidazol) |

Table 3 (11)

| Example | $R^1$ | $R^2$ | n | IR (Nujol) ($cm^{-1}$) | $^1H-NMR$ ($CDCl_3$, δ ppm) |
|---|---|---|---|---|---|
| 44 | 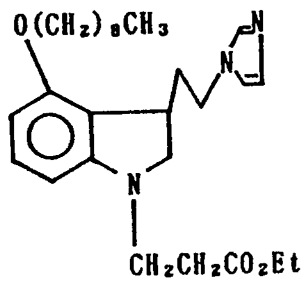 | | | 1735, 1495 | 0.90 (3H, br-t, $CH_2CH_2\underline{CH_3}$), 1.25 (3H, t, J=7Hz, $-OCH_2\underline{CH_3}$), 1.0 ~2.4 (16H, m, $(\underline{CH_2})_{10}$, $C_3-CH_2$), 2.55 (2H, m, J=6Hz, $-CH_2-CO$), 2.8 ~3.6 (5H, m, $C_2-H_2$, $C_3-H$, $N\underline{CH_2}CH_2CO$), 3.98 (2H, br-t, $C_4-O\underline{CH_2}CH_2$), 3.8 ~4.2 (2H, m, $CH_2N$), 4.15 (2H, q, J=7Hz, $-O\underline{CH_2}CH_3$), 6.1 ~6.6 (2H, m, $C_5-H$, $C_7-H$), 6.8 ~7.4 (H, m, $C_6-H$), 6.95, 7.08, 7.48 (3H, s, imidazol) |

Table 3 (12)

| Example | $R^1$ | $R^2$ | n | I R (Nujol) ( $cm^{-1}$) | $^1H-NMR$ ( $CDCl_3$, $\delta$ ppm ) |
|---|---|---|---|---|---|
| 45 | 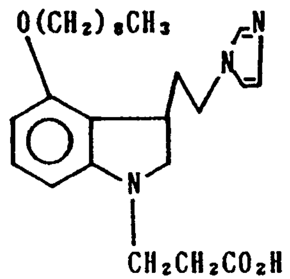 | | | 1700, 1495 | 0.86 (3H, br-t, $CH_2CH_2C\underline{H_3}$), 1.0 ~1.8 (14H, m, $(C\underline{H_2})_7$), 1.8 ~2.3 (2H, m, $C_3-CH_2$), 2.60 (2H, m, $CH_2-CO$), 2.8 ~3.6 (5H, m, $C_2-H_2$, $C_3-H$, $NC\underline{H_2}CH_2CO$), 3.6 ~4.4 (4H, m, $OC\underline{H_2}CH_2$, $CH_2N$), 6.1 ~7.4 (3H, m, phenyl), 6.82, 7.01, 7.69 (3H, s, imidazol) |

Reference Example 1

3-[2-(1H-Imidazol-1-yl)ethyl]-1-indoline

(a) 1-(tert-butoxycarbonyl)-3-(2-hydroxyethyl)-1-indoline

Tryptophole (10.0 g, 62.0 mM) and sodium borohydride (11.73 g, 310.0 mM) are dissolved in pyridine (150 ml), and thereto is split-added alminium chloride (molecular weight 133.34, 24.1 g, 180.6 mM) under ice-cooling. After the addition, the mixture is stirred at 20° C for 8 hours. Water (200 ml) is added thereto, and the insoluble matters are filtered off while washing with benzene (500 ml). The benzene layer is dried over sodium sulfate and the solvent is distilled off under reduced pressure. Thereafter, 6N hydrochloric acid (150 ml) is split-added to decompose pyridine-borane complex. The reaction mixture is made alkaline with sodium carbonate, and extracted with benzene (500 ml). The organic layer is washed with saturated brine (200 ml), dried over sodium sulfate and the solvent is distilled off under reduced pressure. The residue is purified by silica gel column chromatography to give 5.2 g of an oily substance. The oily substance is dissolved in chloroform (40 ml), and added with di-tert-butyldicarbonate (7.65 g, 35 mmol), followed by stirring at room temperature for 2 hours. The chloroform is distilled off under reduced pressure, and the residue is purified by silica gel column chromatography to give 8.10 g of the compound as an oil (Yield 49%).

The properties of the compound thus obtained are as follows.

IR (Neat) $cm^{-1}$ : 3420, 1805

$^1$H-NMR ($CDCl_3$) δ ppm :

1.55 (9H, s, $-CH_3 \times 3$), 1.70 (1H, s, -OH), 1.6 - 2.2 (2H, m, $-CH_2CH_2OH$),

3.2 - 4.2 (5H, m, 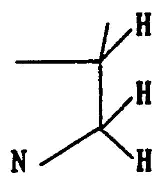 , $-CH_2OH$),

6.1 - 7.8 (4H, m, phenyl)

(b) Title compound

The oily substance from (a) above (40.28 g, 0.153 mol) is dissolved in $CH_3CN$ (700 ml), and carbon tetrabromide (76.1 g, 0.229 M) is added thereto. Triphenylphosphin (48.2 g, 0.184 M) is added thereto at a temperature of 10 - 13° C over 10 minutes. After stirring at room temperature for 1 hour, the $CH_3CN$ is distilled off under reduced pressure, and ethyl acetate (1ℓ ) is added thereto, followed by filtration of the resulting insoluble matters. The filtrate is washed twice with saturated brine (400 ml), dried over anhydrous sodium sulfate and the solvent is distilled off under reduced pressure to give an oily substance, which is dissolved in acetone (1.1ℓ ). Thereto are added imidazole (105 g, 1.53 M) and potassium carbonate (63.5 g, 0.459 M) and the mixture is refluxed for 16 hours. The acetone is distilled off under reduced pressure, and ethyl acetate (1ℓ ) is added thereto, followed by washing with water (700 ml, twice) and with saturated brine (500 ml, once). The organic layer is dried over anhydrous sodium sulfate and the solvent is distilled off under reduced pressure to give 96 g of an oily substance, which is dissolved in 99.5% ethanol (700 ml). Thereto is added 10.0N hydrochloric acid-ethanol (220 ml, 2.20 M) under ice-cooling, and the mixture is stirred at room temperature for 2 hours and at a temperature of 30 - 40° C for 1 hour. The ethanol is concentrated under reduced pressure, and water (600 ml) is added thereto, and the mixture is washed twice with chloroform (600 ml). 4N Sodium hydroxide (ca. 400 ml) is added to adjust the the water layer to pH 8-9, after which the water layer is extracted twice with chloroform (500 ml). The organic layer is washed with saturated brine (500 ml), dried over anhydrous sodium sulfate and the solvent is distilled off under reduced pressure. The precipitated crystals are filtered off with ethyl ether to give 20.09 g of the title compound as a solid (Yield 61.6%). The properties of the compound thus obtained are as follows.

IR (Nujol) $cm^{-1}$ : 3350, 1600, 1510

$^1$H-NMR (DMSO-$D_6$) δ ppm :

1.5 - 2.5 (2H, m, $-CH_2CH_2N<$),

2.7 - 3.7 (3H, m, 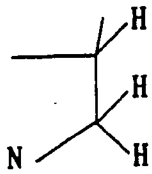 ),

3.6 - 4.6 (3H, m, >NH, $-CH_2N<$),

6.2 - 7.4 (6H, m, phenyl,

(4H, m, phenyl) 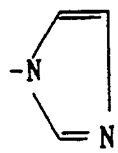 ), 7.65 (1H, s, 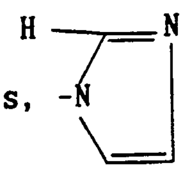 )

Reference Example 2

5-Benzyloxy-3-[2-(1H-imidazol-1-yl)ethyl]indoline hydrochloride

(a) 4-Benzyloxyphenylhydrazine hydrochloride

To 4-benzyloxyaniline hydrochloride (250 g, 1.56 mol) are added water (355 ml) and con. hydrochloric acid (175 ml), and the mixture is stirred while suspending. A solution of $NaNO_2$ (73.2 g, 1.06 mol) in water (180 ml) is dropwise added at a temperature of 0 - 5° C, followed by stirring at the same temperature for 1 hour (A solution).

To con. hydrochloric acid (2.65ℓ ) is added $SnCl_2$ (604 g), and the A solution is added dropwise at a temperature of 0 - 5° C while stirring. After stirring at the same temperature for 30 minutes, $Et_2O$ (2ℓ ) is further added thereto, followed by further stirring for 30 minutes. The precipitated crystals are filtered off, the crystals are added to methanol (3ℓ ), and the mixture is stirred for 30 minutes. The crystals are filtered off, and dried to give 220 g of (a) (Yield 82%).

m.p. 187° C (MeOH-$H_2O$)

TLC (Merck No. 5714 Sol. $CHCl_3$:MeOH = 10:1) Rf = 0.49

IR (Nujol) $cm^{-1}$ : 3230, 1585, 1510

$^1H$-NMR (DMSO-$D_6$) $\delta$ ppm :

5.03 (2H, s, $-CH_2-O$),

6.96 (4H, s, 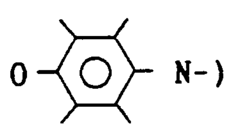 ),

7.35 (5H, s, C-phenyl), 11.0 (3H, br-s, $-NH-NH_2 \cdot HCl$)

(b) 5-Benzyloxytryptophole

4-Benzyloxyphenylhydrazine hydrochloride (340 g, 1.35 mol) is added to methanol (8.3ℓ ), and dihydrofuran (162 ml, ca. 2.14 mol) is added thereto, followed by stirring at 50 ° C for 10 hours while suspending. The solvent is distilled off under reduced pressure, and chloroform (3ℓ ) is added thereto. The insoluble matters are filtered off, and the filtrate is concentrated to give an oily substance which is then purified by silica gel column chromatography (developing solvent; $CHCL_3$: AcOEt:MeOH = 80:40:1 - 40:40:1) to give 170 g of an oily substance

(b) (Yield 46%).
TLC (Merck No. 5714 Sol. $CHCl_3$:AcOEt:MeOH = 10:10:1) Rf = 0.70
IR (Nujol) $cm^{-1}$ : 3420, 1620, 1480
$^1$H-NMR (DMSO-$D_6$) δ ppm :
2.95 (2H, t, J = 6Hz, -$C_3$,-$CH_2$), 3.85 (2H, t, J = 6Hz, -$\underline{CH_2}$OH), 5.07 (2H, s, O$\underline{CH_2}$ φ ), 6.7 - 7.6 (9H, m, $C_2$-H, phenyl), 7.95 (1H, br-s, -NH)

(c) 3-Acetoxyethyl-5-benzyloxy-1-(tert-butoxycarbonyl)indoline

Pyridine (20 ml) is added to 5-benzyloxytryptophole (170 g), and $Ac_2O$ (200 ml) is dropwise added thereto. The mixture is reacted at room temperature for 1 hour, extracted with AcOEt (2 ℓ ), and washed with water. Thereafter, the mixture is further washed with an aqueous solution of $NaHCO_3$ and an aqueous solution of NaCl, and dried over $Na_2SO_4$. The AcOEt is distilled off under reduced pressure and isopropyl ether (700 ml) is added to the residue, followed by stirring. The precipitated crystals are filtered off and dried to give 168 g of acetate (Yield 85%).
TLC (Merck No. 5714 Sol. Benzene:AcOEt = 5:1) Rf = 0.60
IR (Nujol) $cm^{-1}$ : 1735, 1625, 1492
$^1$H-NMR ($CDCl_3$) δ ppm :
2.02 (3H, s, $OCOCH_3$), 3.01 (2H, t, J = 6Hz, $C_3$-$CH_2$), 4.30 (2H, t,J = 6Hz, $\underline{CH_2}$OAc), 5.10 (2H, s, -$OCH_2$ φ ), 6.7 - 7.6 (9H, m, $C_2$-H, $C_4$-H, $C_6$-H, $C_7$-H, φ ) 7.85 (1, br, -NH)

The acetate (79 g, 0.255 mol) is suspended in AcOH (520 ml), and split-added with $NaCNBH_3$ (85 g, 1.35 mol) at 15 °C. After stirring at the same temperature for 2 hours, the mixture is poured into ice-water, added with 4N-NaOH (ca. 2ℓ ) to adjust the mixture to pH 8, and extracted with $CH_2Cl_2$ (1ℓ ). The extract is washed with an aqueous solution of NaCl, dried over $Na_2SO_4$, and concentrated till the $CH_2Cl_2$ amount becomes 800 ml. Thereto is added $(Boc)_2O$ (64 g, 0.293 mol) at 5 °C, and the mixture is stirred at room temperature for 14 hours. The reaction mixture is washed with an aqueous solution of 1N-NaOH and an aqueous solution of NaCl, and dried over $Na_2SO_4$. The solvent is distilled off under reduced pressure, and the residue is purified by silica gel column chromatography (developing solvent; n-hexane:AcOEt = 10:1 - 3:1) to give 92.9 g of a solid (c) (Yield 88%).
TLC (Merck No. 5714 Sol. Benzene:AcOEt = 1:1) Rf = 0.48
IR (Nujol) $cm^{-1}$ : 1740, 1695, 1492
$^1$H-NMR ($CDCl_3$) δ ppm :
1.45 (9H, s, -$C(CH_3)_3$), 2.0 (2H, m, $C_3$-$CH_2$), 2.9 - 4.2 (3H, m, $C_2$-$H_2$, $C_3$-H), 4.12 (2H, t, J = 6Hz, $CH_2$OAc), 4.97 (2H, s, -O$\underline{CH_2}$ φ ), 6.75 (2H, m, $C_4$-H, $C_6$-H), 7.41 (5H, St $OCH_2$ φ ), 7.60 (1H, m, -$C_7$-H)

(d) 5-Benzyloxy-1-(tert-butoxycarbonyl)-3-hydroxyethylindoline

The solid (c) (187 g, 0.455 mol) is dissolved in 95% EtOH (1000 ml), and NaOH (57 g, 1.42 mol) is added thereto. After stirring the mixture at room temperature for 3 hours, the solvent is distilled off. The mixture is extracted with $CHCl_3$, washed with water, dried over $Na_2SO_4$, and the solvent is distilled off under reduced pressure. To the oily substance thus obtained is added isopropyl ether (800 ml) and seeds, followed by stirring. The precipitated crystals are filtered off and dried to give 159 g of (d) (Yield 94%).
TLC (Merck No. 5714 Sol. n-Hexane:AcOEt = 2:1) Rf = 0.29
IR (Nujol) $cm^{-1}$ : 3320, 1705, 1490
$^1$H-NMR ($CDCl_3$) δ ppm :
1.46 (9H, s, -$C(CH_3)_3$), 1.90 (2H, m, $C_3$-$CH_2$), 3.0 - 4.2 (6H, m, $C_2$-$H_2$, $C_3$-H, $CH_2$OH), 4.12 (2H, t, J = 6Hz, $CH_2$OAc), 4.98 (2H, s, -O$\underline{CH_2}$ φ ), 6.5 - 7.8 (3H, m, $C_4$-H, $C_6$-H, -$C_7$-H), 7.32 (5H, s, $OCH_2$ φ )

(e) Title compound

3-Hydroxyethylindoline (d) (80 g, 0.217 mol) is dissolved in $CH_3CN$ (1.6ℓ ), and thereto is added $CBr_4$ - (107.6 g, 0.324 mol), followed by split-addition of $(C_6H_5)_3P$ (85.2 g, 0.324 mol) under ice-cooling. The mixture is stirred at the same temperature for 20 minutes and at room temperature for 1 hour. After the $CH_3CN$ is distilled off under reduced pressure, the mixture is extracted with $CHCl_3$, washed with water, dried over $Na_2SO_4$, and the solvent is distilled off under reduced pressure. The oily substance thus

obtained is dissolved in acetone (2.6ℓ), added with imidazole (150 g) and $K_2CO_3$ (91 g) and refluxed for 18 hours. The solvent is distilled off under reduced pressure, and the mixture is extracted with $CH_2Cl_2$, washed 3 times with water (400 ml) and NaCl, and dried over $Na_2SO_4$, after which the solvent is distilled off under reduced pressure. The oily substance thus obtained is dissolved in 99.5% EtOH (460 ml), added with 10N-HCl/EtOH (460 ml) under ice-cooling, and the mixture is stirred at room temperature for 2 hours. The EtOH is distilled off under reduced pressure and the residue thus obtained is extracted to give 63 g of (e) as crude crystals (Yield 74%). The crude crystals are recrystallized from 99.5% EtOH to give 43 g of the compound as crystals, m.p. 210° C.
TLC (Merck No. 5714 Sol. $CHCl_3$:MeOH = 8:1) Rf = 0.24

[Formulation Examples]

Formulation 1
Tablets

| | |
|---|---|
| Compound of the invention | 100 mg |
| Abysel | 35 mg |
| Lactose | 20 mg |
| Corn starch | 18 mg |
| Talc | 5 mg |
| Magnesium stearate | 2 mg |
| Total amount | 180 mg |

According to the above composition, an aqueous solution of corn starch is added to a mixture of the compound of the present invention, abysel and lactose, mixed in advance. The mixture is kneaded, dried and pulverized to give whole grains. To the obtained whole grains is added a mixture of talc and magnesium stearate and the mixture is further mixed, which is then pounded to prepare tablets.

Formulation 2
Capsules

| | |
|---|---|
| Compound of the invention | 50 mg |
| Lactose | 68 mg |
| Corn starch | 25 mg |
| Polyvinylpyrrolidone | 5 mg |
| Stearic acid | 2 mg |
| Total amount | 150 mg |

According to the above composition, an alcohol solution of polyvinylpyrrolidone and stearic acid is added to a mixture of the compound of the present invention, lactose and corn starch, mixed in advance. The mixture is kneaded and granulated to give granules. The obtained granules are dried and filled in capsules No. 3 to prepare capsules.

Formulation 3
Injections (water soluble agents)

In 2-ml ampoules,

| | |
|---|---|
| Compound of the invention | 20 mg |

Distilled water for injection in an amount to make the total amount 2 ml.

Formulation 4
Injections (solid agents)

In 1 vial,

| | |
|---|---|
| Compound of the invention | 30 mg |
| Mannitol | 100 mg |

## Claims

1. An imidazole derivative of the formula

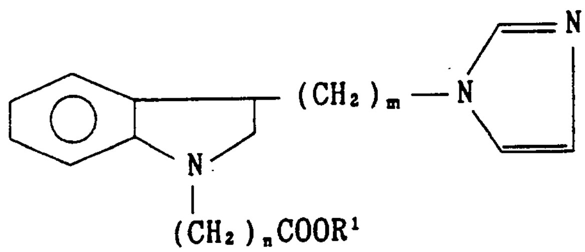

(I)

wherein m and n are respectively an integer from 1 to 3, $R^1$ is a hydrogen atom or an ester residue, and at least one substituent selected from alkyls having 1 to 10 carbon atoms and alkoxys having 1 to 15 carbon atoms may be comprised on the indoline skeleton or a pharmacologically acceptable salt thereof.

2. An imidazole derivative or a pharmacologically acceptable salt thereof as claimed in Claim 1, wherein the alkyl substituted on the indoline skeleton has 3 to 8 carbon atoms.

3. An imidazole derivative or a pharmacologically acceptable salt thereof as claimed in Claim 1, wherein the alkoxy substituted on the indoline skeleton has 5 to 9 carbon atoms.

4. An imidazole derivative or a pharmacologically acceptable salt thereof as claimed in Claim 1, which is selected from the following compounds.

- o Ethyl 3-[2-(1H-imidazol-1-yl)ethyl]-5-methoxy-1-indolinepropionate
- o Ethyl 3-[2-(1H-imidazol-1-yl)ethyl]-5-pentyloxy-1-indolinepropionate oxalate
- o 3-[2-(1H-Imidazol-1-yl)ethyl]-5-pentyloxy-1-indolineacetic acid
- o Ethyl 3-[2-(1H-imidazol-1-yl)ethyl]-5-pentyloxy-1-indolineacetate
- o 3-[2-(1H-Imidazol-1-yl)ethyl]-5-pentyloxy-1-indolinepropionic acid
- o 5-Heptyloxy-3-[2-(1H-imidazol-1-yl)ethyl]-1-indolinepropionic acid
- o Ethyl 5-heptyloxy-3-[2-(1H-imidazol-1-yl)ethyl]-1-indolinepropionate oxalate
- o 5-Hexyloxy-3-[2-(1H-imidazol-1-yl)ethyl]-1-indolinepropionic acid
- o Ethyl 5-hexyloxy-3-[2-(1H-imidazol-1-yl)ethyl]-1-indolinepropionate
- o 3-[2-(1H-Imidazol-1-yl)ethyl]-5-octyloxy-1-indolinepropionic acid
- o Ethyl 3-[2-(1H-imidazol-1-yl)ethyl]-5-octyloxy-1-indolinepropionate oxalate
- o 3-[2-(1H-Imidazol-1-yl)ethyl]-5-nonyloxy-1-indolinepropionic acid
- o Ethyl 3-[2-(1H-imidazol-1-yl)ethyl]-5-nonyloxy-1-indolinepropionate oxalate
- o 3-[2-(1H-Imidazol-1-yl)ethyl]-5-hexyl-1-indolinepropionic acid
- o Ethyl 3-[2-(1H-imidazol-1-yl)ethyl]-5-hexyl-1-indolinepropionate

5. A method for producing an imidazole derivative or a pharmacologically acceptable salt thereof as claimed in Claim 1, comprising ① reacting a compound (II) of the formula

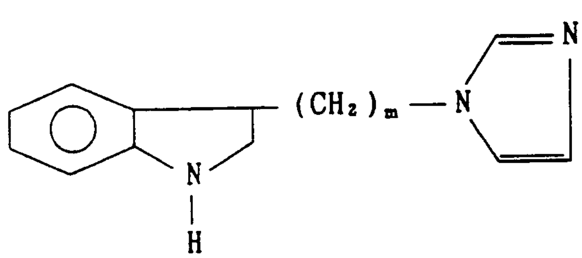

(II)

wherein m is an integer from 1 to 3, and a compound (III) of the formula

$$X-(CH_2)_nCOOR^1 \qquad (III)$$

wherein n is an integer from 1 to 3, X is a group reactive with the amino group and $R^1$ is H or an alkyl group, ② reacting the compound (II) and a compound of the formula

$$CH_2=CH-COOR^1 \qquad (VIII)$$

wherein $R^1$ is as defined above, ③ reacting a compound (X) of the formula

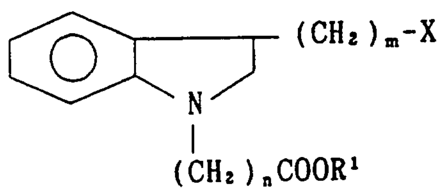

(X)

wherein n, m and $R^1$ are as defined above, and imidazole, or ④ reacting a compound (XI) of the formula

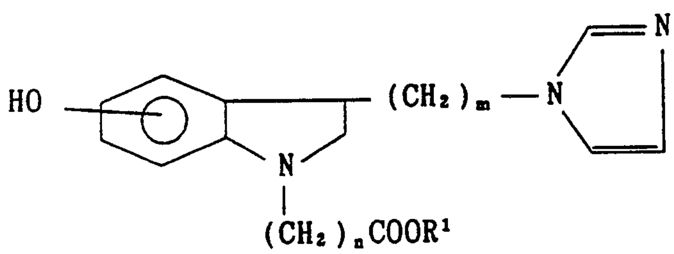

(XI)

wherein m, n, $R^1$ and X are as defined above, and a compound (X II ) of the formula

$$R_2-Y \qquad (XII)$$

wherein Y is a group reactive with the alkoxy group and $R_2$ is an alkyl group.

6. A pharmaceutical composition containing an imidazole derivative or a pharmacologically acceptable salt thereof as claimed in Claim 1 as an active ingredient.

7. A pharmaceutical composition as claimed in Claim 5, which is an agent for the prevention and treatment of the diseases caused by thromboxane $A_2$.

8. A pharmaceutical composition as claimed in Claim 5, which is an agent for the prevention and treatment of the diseases caused by lipoperoxide.

9. A pharmaceutical composition as claimed in Claim 5, which comprises at least one compound selected from the following compounds and a carrier.
   o Ethyl 3-[2-(1H-imidazol-1-yl)ethyl]-5-methoxy-1-indolinepropionate
   o 3-[2-(1H-Imidazol-1-yl)ethyl]-5-pentyloxy-1-indolineacetic acid
   o Ethyl 3-[2-(1H-imidazol-1-yl)ethyl]-5-pentyloxy-1-indolineacetate

- Ethyl 3-[2-(1H-imidazol-1-yl)ethyl]-5-pentyloxy-1-indolinepropionate oxalate
- 3-[2-(1H-Imidazol-1-yl)ethyl]-5-pentyloxy-1-indolinepropionic acid
- 5-Heptyloxy-3-[2-(1H-imidazol-1-yl)ethyl]-1-indolinepropionic acid
- Ethyl 5-heptyloxy-3-[2-(1H-imidazol-1-yl)ethyl]-1-indolinepropionate oxalate
- 5-Hexyloxy-3-[2-(1H-imidazol-1-yl)ethyl]-1-indolinepropionic acid
- Ethyl 5-hexyloxy-3-[2-(1H-imidazol-1-yl)ethyl]-1-indolinepropionate
- 3-[2-(1H-Imidazol-1-yl)ethyl]-5-octyloxy-1-indolinepropionic acid
- Ethyl 3-[2-(1H-imidazol-1-yl)ethyl]-5-octyloxy-1-indolinepropionate oxalate
- 3-[2-(1H-Imidazol-1-yl)ethyl]-5-nonyloxy-1-indolinepropionic acid
- Ethyl 3-[2-(1H-imidazol-1-yl)ethyl]-5-nonyloxy-1-indolinepropionate oxalate
- 3-[2-(1H-Imidazol-1-yl)ethyl]-5-hexyl-1-indolinepropionic acid
- Ethyl 3-[2-(1H-imidazol-1-yl)ethyl]-5-hexyl-1-indolinepropionate

# INTERNATIONAL SEARCH REPORT

International Application No PCT/JP90/00409

**I. CLASSIFICATION OF SUBJECT MATTER** (if several classification symbols apply, indicate all) [6]

According to International Patent Classification (IPC) or to both National Classification and IPC

Int. $Cl^5$ C07D403/06, A61K31/415

**II. FIELDS SEARCHED**

Minimum Documentation Searched [7]

| Classification System | Classification Symbols |
|---|---|
| IPC | C07D403/02, 403/06, A61K31/415 |

Documentation Searched other than Minimum Documentation to the Extent that such Documents are Included in the Fields Searched [8]

**III. DOCUMENTS CONSIDERED TO BE RELEVANT** [9]

| Category * | Citation of Document, [11] with indication, where appropriate, of the relevant passages [12] | Relevant to Claim No. [13] |
|---|---|---|
| Y | US, A, 4273782 (Pfizer Inc), 16 June 1981 (16. 06. 81) & JP, A, 55-133380 | 1 - 9 |
| A | EP, A, 69513 (Pfizer Inc), 12 January 1983 (12. 01. 83) & JP, A, 58-41875 | 1 - 9 |
| A | EP, A, 3901 (Pfizer Inc), 5 September 1979 (05. 09. 79) & JP, A, 54-132569 | 1 - 9 |
| A | EP, A, 73663 (Pfizer Inc), 9 March 1983 (09. 03. 83) & JP, A, 58-52272 | 1 - 9 |

* Special categories of cited documents: [10]

"A" document defining the general state of the art which is not considered to be of particular relevance

"E" earlier document but published on or after the international filing date

"L" document which may throw doubts on priority claim(s) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step

"Y" document of particular relevance; the claimed invention cannot be considered to involve an inventive step when the document is combined with one or more other such documents, such combination being obvious to a person skilled in the art

"&" document member of the same patent family

**IV. CERTIFICATION**

| Date of the Actual Completion of the International Search | Date of Mailing of this International Search Report |
|---|---|
| June 6, 1990 (06. 06. 90) | June 18, 1990 (18. 06. 90) |
| **International Searching Authority** | **Signature of Authorized Officer** |
| Japanese Patent Office | |